# EUROPEAN PATENT SPECIFICATION

(11) **EP 4 141 107 B1**
(45) Date of publication and mention of the grant of the patent: **11.03.2026**
(21) Application number: 21793580.8
(22) Date of filing: 23.04.2021
(51) Int. Cl.: C12N 5/078, B01D 15/38, B01J 20/28, B01J 20/286, B01J 20/32, G01N 33/50, G01N 33/543

(54) **MICROVESICLE ISOLATION METHOD AND MICROVESICLE ISOLATION DEVICE**
MIKROVESIKELISOLIERUNGSVERFAHREN UND MIKROVESIKELISOLIERUNGSVORRICHTUNG
PROCÉDÉ D'ISOLEMENT DE MICROVÉSICULE ET DISPOSITIF D'ISOLATION DE MICROVÉSICULE

(30) Priority: 24.04.2020 KR 20200050314; 22.04.2021 KR 20210052200
(43) Date of publication of application: 01.03.2023
(73) Proprietor: Korea University Research and Business Foundation, Seoul 02841 (KR)
(72) Inventor: SHIN, Se-Hyun, Seoul 06629 (KR); KIM, Hyun-Sung, Seoul 06318 (KR)
(74) Representative: Meissner Bolte Partnerschaft mbB
(86) International application number: PCT/KR2021/005178
(87) International publication number: WO 2021/215878

(56) References cited:
- WO-A1-2014/107571
- WO-A1-2017/198695
- WO-A1-2019/039179
- CN-A- 108 841 777
- KR-A- 20170 028 432
- KR-A- 20190 012 130
- KIM JINHYUN, LEE HOYOON, PARK KYONGHWA, SHIN SEHYUN: "Rapid and Efficient Isolation of Exosomes by Clustering and Scattering", JOURNAL OF CLINICAL MEDICINE, vol. 9, no. 650, pages 1 - 15, XP055860901, DOI: 10.3390/jcm9030650

## Description

### FIELD

The present invention relates to a microvesicles isolation method and a microvesicles isolation device from a biological sample.

### DESCRIPTION OF RELATED ART

Microvesicles are a type of a membrane vesicle released from a cell, and include exosomes or extracellular vesicles. The microvesicles from various cellular sources share characteristics of a parental cell and thus are attracting attention as a novel diagnostic marker of diseases. However, the microvesicles has a very small size of 50 to 200 nm such that it is it difficult to extract the microvesicle.

Conventional techniques for isolating the extracellular vesicle include ultra-centrifugation isolation, size exclusion, immunoaffinity isolation, microfluidics chip, and polymeric method. etc. Among them, the ultra-centrifugation isolation is the most widely used method to isolate the extracellular vesicles, and is the most reliable isolation method due to its simple principle.

However, when isolating the microvesicles using such ultra-centrifugation isolation, a yield of the microvesicles is low, and it takes a lot of time to isolate the same (8 hours or more). Further, the ultra-centrifugation isolation requires an expert in extraction, and requires expensive equipment and thus is disadvantageous in terms of cost.

The size exclusion is mainly used together with the ultra-centrifugation isolation method, and has the advantage of simply increasing a purity of the microvesicles, but has a limitation in that the yield is low because the microvesicles stick to a filter.

The immunoaffinity isolation includes attaching an antibody to the extracellular vesicle and thus may isolate a specific microvesicles at very high specificity. However, there is a limitation in that the extraction efficiency is low because there is no universally usable antibody for general microvesicles. Further, since an antibody making process takes a long time and costs a lot, this scheme is not suitable for practical diagnosis.

Further, the polymeric method is a technology of adding PEG (polyethylene glycol) to a sample to lower a solubility to cause the microvesicles to settle down. This scheme is still a precipitation method using a centrifugal separator, so that a detection cost is expensive, a long incubation time is required, and a purity of the precipitate is not good because the microvesicles together with the protein settle down. Thus, this scheme is not suitable for use for diagnosis.

An existing scheme for isolating the microvesicles using electrical charge characteristics is also disclosed. However, in a sample such as plasma, many plasma proteins such as fibrinogen have a zeta potential similar to that of the microvesicles. Thus, it is being reported that it is somewhat difficult to extract the microvesicles only using an isoelectric point.

### (Prior literature)

Korean Patent Application Publication No. 10-2005-0119128 (December 20, 2005) and CN 108 841 777 A.

### DISCLOSURE

### TECHNICAL PURPOSE

A purpose of the present disclosure is to provide a microvesicles isolation method and a microvesicles isolation device that may quickly and easily obtain microvesicles from a biological sample without centrifugal isolation.

Further, another purpose of the present disclosure is to provide a microvesicles isolation method and a microvesicles isolation device that may isolate microvesicles contained in the biological sample at a high isolation efficiency and may not require additional post-processing.

### TECHNICAL SOLUTION

According to one aspect of the present invention, embodiments of the present invention include a microvesicles isolation method and a microvesicles isolation device. Hereinafter, the terminology "embodiment" relates to the disclosure and only forms part of the invention if it falls within the scope of the claims.

The invention pertains to a microvesicles isolation method to isolate microvesicles contained in the biological sample from the sample, the method comprising:
(a) adding an adsorbent sphere to the biological sample containing the microvesicles therein;
(b) keeping the adsorbent sphere in the biological sample to form an adsorbent sphere conjugate composed of the adsorbent sphere and the microvesicles captured thereon;
(c) isolating the adsorbent sphere conjugate from the biological sample;
(d) washing the isolated adsorbent sphere conjugate using a first reagent, including washing the adsorbent sphere having the microvesicles captured thereon under a first condition using the first reagent,
   wherein the first reagent contains at least one of CH3COO-, SO42-, HCO-, SiO-, and OH-, wherein the first condition is that the washing is carried out at least once in a range of pH 5.5 to 6.5 or pH 7.5 to 9.5; and
(e) eluting the microvesicles from the washed adsorbent sphere conjugate using a second reagent, wherein the adsorbent sphere includes a support, and one or more polyvalent cations disposed on a surface of the support.

The invention further pertains to a reagent kit for microvesicles isolation configured to isolate the microvesicles contained in a biological sample, the reagent kit comprising:
one or more adsorbent spheres having binding ability to the microvesicle; a first reagent containing at least one of CH3COO-, SO42-, HCO-, SiO-, and OH- and having a pH in the range of pH 5.5 to 6.5 or pH 7.5 to 9.5; and a second reagent containing at least one of CH3COO-, SO42-, Cl-, HCO-, SiO-, and OH- , wherein the adsorbent sphere includes a support, and one or more polyvalent cations disposed on a surface of the support.

In an exemplary embodiment, the biological sample may include at least one of blood, plasma, serum, urine, saliva, cerebrospinal fluid, tears, sweat, feces, ascites, amniotic fluid, semen, milk, cell medium, tissue extract and cancer tissue.

In an exemplary embodiment, the support may include at least one of polyacrylate, polyacrylamide, polymethacrylate, polyethylene glycol, polystyrene vinylbenzene, polystyrene, hydrogel, agarose, ceramic, silica gel, latex, metal particles, glass particles, magnetic particles, metal wires, or metal wires having magnetic nanoparticles bonded thereto.

In an exemplary embodiment, the polyvalent cation may include at least one of polylysine, protamine, polyarginine, polyhistidine, cationic dextran, cationic dendrimer, cationic polysaccharide, polyamidoamine, polyethyleneimine, polyquaternium, poly-2-dimethylaminoethyl methacrylate (PDMAEMA), poly (2-dimethylaminomethyl styrene) (PDMAMS), poly-1-vinylpyrrolidone (p1-VP), poly diethylaminoethyl acrylate (pDEAEA), poly dimethylaminoethyl acrylate (pDMAEA), poly diethylaminoethyl methacrylate (pDEAMA), lipopolyamines, quaternary ammoniums, guanidine, imidazole, polyaniline, polypyrrol, or chitosan.

In an exemplary embodiment, the support may include a spherical shape, and an average diameter of the support is in a range of 200 nm inclusive to 100 µm inclusive.

In an exemplary embodiment, the support may include at least one of a porous particle, a porous membrane, or a porous mesh.

In an exemplary embodiment, the support may include irregularities formed on the surface thereof.

In an exemplary embodiment, the support may be a magnetic bead containing at least one metal selected from a group consisting of iron (Fe), nickel (Ni), cobalt (Co), manganese (Mn), bismuth (Bi) and zinc (Zn), or an alloy thereof, or a metal oxide or alloy oxide of a metal selected from the group.

In an exemplary embodiment, the support may include a core as an inner portion thereof, and a shell provided to surround an outer face of the core, wherein the core includes at least one of polyacrylate, polyacrylamide, polymethacrylate, polyethylene glycol, polystyrene vinylbenzene, polystyrene, hydrogel, agarose, ceramic, silica gel, or latex, wherein the shell contains at least one metal selected from a group consisting of iron (Fe), nickel (Ni), cobalt (Co), manganese (Mn), bismuth (Bi) and zinc (Zn), or an alloy thereof, or a metal oxide or alloy oxide of a metal selected from the group, wherein the polyvalent cation includes at least one of poly-L-lysine polymer (PLL), protamine, quaternary ammoniums, chitosan, or polyhistidine.

In an exemplary embodiment, the support may include a core as an inner portion thereof, and a shell provided to surround an outer face of the core, wherein the core contains at least one metal selected from a group consisting of iron (Fe), nickel (Ni), cobalt (Co), manganese (Mn), bismuth (Bi) and zinc (Zn), or an alloy thereof, or a metal oxide or alloy oxide of a metal selected from the group, wherein the shell includes at least one of polyacrylate, polyacrylamide, polymethacrylate, polyethylene glycol, polystyrene vinylbenzene, polystyrene, hydrogel, agarose, ceramic, silica gel, or latex, wherein the polyvalent cation includes at least one of poly-L-lysine polymer (PLL), protamine, quaternary ammoniums, chitosan, or polyhistidine.

In an exemplary embodiment, in the step (b), the keeping of the adsorbent sphere in the biological sample may include stirring the adsorbent sphere at a temperature of 1°C to 8°C, for 10 minutes to 2 hours, and at 50rpm to 150rpm.

In an exemplary embodiment, in the step (c), the adsorbent sphere conjugate may be isolated using a isolation member, wherein the isolation member includes at least one of a capturing filter, magnetic isolation, centrifugal isolation, solubility-based isolation, or isolation based on a particle size.

In an exemplary embodiment, the step (d) may include washing the adsorbent sphere having the microvesicles captured thereon under a first condition using the first reagent, wherein the first reagent contains at least one of CH₃COO⁻, SO₄²⁻, HCO⁻, SiO⁻, and OH⁻, wherein the first condition is that the washing is carried out at least once in a range of pH 5.5 to 6.5, and/or pH 7.5 to 9.5.

In an exemplary embodiment, the step (e) may include eluting the microvesicles from the washed adsorbent sphere conjugate using the second reagent under a second condition, wherein the second reagent contains at least one of CH₃COO⁻, SO₄²⁻, Cl⁻, HCO⁻, SiO⁻, and OH⁻ at a concentration range of 0.2 M to 3 M, wherein the second condition is that the elution is performed at least once via mixing at 500rpm to 2000rpm and for 1 minute to 60 minutes.

In an exemplary embodiment, the method may further comprise: after washing the adsorbent sphere conjugate with the first reagent in the step (d), performing a first auxiliary isolation to isolate the adsorbent sphere conjugate; and after the elution of the microvesicles with the second reagent in the step (e), performing a second auxiliary isolation to isolate the adsorbent sphere, wherein the support is embodied as a bead having a circular cross section and made of at least one of polyacrylate, polyacrylamide, polymethacrylate, polyethylene glycol, polystyrene vinylbenzene, or polystyrene, wherein the first auxiliary isolation or the second auxiliary isolation is performed using a capturing filter having pores of 50 nm to 1,000 nm in size.

In an exemplary embodiment, the method may further comprise: after washing the adsorbent sphere conjugate with the first reagent in the step (d), performing a first auxiliary isolation to isolate the adsorbent sphere conjugate; and after the elution of the microvesicles with the second reagent in the step (e), performing a second auxiliary isolation to isolate the adsorbent sphere, wherein the support includes a magnetic particle, wherein the first auxiliary isolation or the second auxiliary isolation includes magnetic isolation to cause current to flow in an electromagnet to generate a magnetic force or using a magnet.

In an exemplary embodiment, the method may further comprise pre-treating the biological sample before the adsorbent sphere is added thereto, wherein the pre-treatment of the biological sample includes filtering the biological sample so that a substance having an average diameter of 0.8 µm or greater is isolated from the biological sample.

In an exemplary embodiment, the microvesicles may be coupled to the adsorbent sphere via at least one of electrostatic interaction or intercalation, wherein a zeta potential of the adsorbent sphere conjugate is in a range of 0.5mV to 10mV.

In an exemplary embodiment, each of the microvesicles captured onto the adsorbent sphere may include an average diameter in a range of 20 nm to 800 nm, wherein the polyvalent cation includes at least one of poly-L-lysine polymer (PLL), protamine, quaternary ammoniums, chitosan, or polyhistidine.

Another exemplary embodiment of the present invention provides a reagent kit for microvesicles isolation configured to isolates the microvesicles contained in a biological sample, the reagent kit comprising: one or more adsorbent spheres having binding ability to the microvesicle; a first reagent containing at least one of CH₃COO⁻, SO₄²⁻, HCO⁻, SiO⁻, and OH⁻; and a second reagent containing at least one of CH₃COO⁻, SO₄²⁻, Cl⁻, HCO⁻, SiO⁻, and OH⁻ , wherein the adsorbent sphere includes a support, and one or more polyvalent cations disposed on a surface of the support.

In an exemplary embodiment, the reagent kit may further comprise an isolation member for isolating the adsorbent sphere or the adsorbent sphere having the microvesicle captured thereon, wherein the isolation member includes at least one of a capturing filter, magnetic isolation, centrifugal isolation, solubility-based isolation, or isolation based on a particle size.

In an exemplary embodiment, the support may be embodied as a bead having a circular cross section and made of at least one of polyacrylate, polyacrylamide, polymethacrylate, polyethylene glycol, polystyrene vinylbenzene, or polystyrene, wherein the polyvalent cation includes at least one of poly-L-lysine polymer (PLL), protamine, quaternary ammoniums, chitosan, or polyhistidine, wherein the isolation member includes a capturing filter having pores of 50 nm to 1,000 nm in size.

In an exemplary embodiment, the support includes a magnetic particle, wherein the polyvalent cation includes at least one of poly-L-lysine polymer (PLL), protamine, quaternary ammoniums, chitosan, or polyhistidine, wherein the isolation member performs magnetic isolation using a device to cause a current to flow in an electromagnet to generate a magnetic force or using a magnet.

### TECHNICAL EFFECT

According to the present disclosure, the microvesicles isolation method and the microvesicles isolation device may isolate the microvesicles from the biological sample at a high yield and at a high purity in a short period of time without using the expensive equipment which is required in the centrifugal isolation, and thus may be usefully used for on-site diagnosis, etc. where a bulky device such as a centrifugal isolation device cannot be placed.

Further, the present disclosure may provide the microvesicles isolation method and the microvesicles isolation device that may isolate the microvesicles at a very high purity while using a small volume of the biological sample.
FIG. 1 is a flowchart showing a microvesicles isolation method according to an embodiment of the present disclosure.
FIG. 2 is a diagram schematically showing an adsorbent sphere according to an embodiment of the present disclosure.
FIG. 3A is a diagram schematically showing an adsorbent sphere according to another embodiment of the present disclosure.
FIG. 3B is a diagram schematically showing support according to still another embodiment of the present disclosure.
FIG. 4 schematically shows the microvesicles isolation method according to Present Example 1.
FIG. 5 shows a result of identifying the isolation ability of extracellular vesicles using the PLL-bead according to Present Example 1 and the bare-bead as a bead to which a cationic polymer is not bound.
FIG. 6 is a diagram schematically showing how the PLL-bead of Present Example 1.
FIG. 7 shows a fluorescent image of the PLL-bead as prepared in a manner according to FIG. 6
FIG. 8 shows the results of identifying performances based on various first reagents using Present Example 1.
FIG. 9 shows the results of identifying the performance based on the first reagent to which the salt was added, using Present Example 1.
FIG. 10 shows results of identifying the performances based on various second reagents using Present Example 1.
FIG. 11 is a graph showing a purity related to the particles and proteins contained in the second reagent, based on the type of the first reagent.
FIG. 12 shows the results of evaluating the conventional centrifugal isolation method and the isolation method according to Present Example 1 of the present disclosure.
FIG. 13 is a diagram schematically showing the microvesicles isolation method according to Present Example 2.
FIG. 14 shows the result of evaluating the performance of the PLL-magnetic beads based on a PLL concentration, an incubation temperature, and an incubation time.
FIG. 15 shows the result of evaluating the extracellular vesicle isolation ability based on the size of the PLL-magnetic bead.
FIG. 16 shows the SEM image, the fluorescence image, and the cryo-TEM image according to each of Present Example 2 and Comparative Example 2.
FIG. 17 shows the results of identifying the zeta potential and the volume change after incubation according to Present Example 2.
FIG. 18 shows the results of NTA and BCA analysis of each of Present Example 2 and Comparative Example 2 to Comparative Example 4.
FIG. 19 shows the analysis results of NTA and BCA according to the first reagent of each of Present Example 2 and Present Example 3.
FIG. 20 shows the Western blot result of each of Present Example 2 and Comparative Example 2 to Comparative Example 4.
FIG. 21 shows the performance evaluation result using a bioanalyzer of each of Present Example 2 and Comparative Example 2 to Comparative Example 4, and an exosome miRNA measurement result using RT-qPCR of each of Present Example 2 and Comparative Example 2 to Comparative Example 4.
FIG. 22 shows the result of exosome miRNA measurement using RT-qPCR about each of Present Example 2 and Present Example 3.
FIG. 23 is a graph depicting gene expression using a microarray according to each of Present Example 2 and Comparative Example 2 to Comparative Example 4.
FIG. 24 is the result of using micro-array according to Present Example 3.
FIG. 25 shows the results of performance evaluation in the step (a) of using the first reagent and the step (b) of using the second reagent in Present Example 2.

Specific details of other exemplary embodiments are included in the detailed description and drawings.

The benefits and features of the present application, and the methods of achieving the benefits and features will become apparent with reference to embodiments to be described below in detail along with the accompanying drawings. However, the present invention is limited by the appended claims only, and not limited to the embodiments disclosed below and may be implemented in various different forms, and unless otherwise specified in the following description, all numbers, values and/or expressions expressing ingredients, reaction conditions, and contents of ingredients in the present invention are to be understood as modified in all instances by the term "about" as such numbers are inherently approximations that are reflective of, among other things, the various uncertainties of measurement encountered in obtaining such values. In addition, when a numerical range is disclosed in the present description, the numerical range is continuous, and includes, unless otherwise indicated, every value up to a maximum value, which includes a minimum value to the maximum value of the numerical range. Furthermore, when the numerical range refers to integers, unless otherwise indicated, the integers include every integer up to a maximum value, which includes a minimum value to the maximum value.

Further, it will be appreciated that in the present invention, when a range is described for a variable, the variable includes all the values in the described range including the end points described in the range. It will be appreciated that for example, a range of "5 to 10" includes not only values of 5, 6, 7, 8, 9, and 10, but also any sub range of 6 to 10, 7 to 10, 6 to 9, 7 to 9, and the like, and also includes any value between appropriate integers within the described range, such as 5.5, 6.5, 7.5, 5.5 to 8.5, and 6.5 to 9. It will be appreciated that for example, a range of "10% to 30%" includes not only all the integers including values of 10%, 11%, 12%, 13%, and the like and up to 30%, but also any sub range of 10% to 15%, 12% to 18%, 20% to 30%, and the like, and also includes any value between appropriate integers within the described range, such as 10.5%, 15.5%, and 25.5%.

In the present disclosure, the term 'microvesicles' is used synonymously with the extracellular vesicle released from the cell membrane of Archaea, Prokarya or Eukarya. In the present disclosure, the microvesicles may include exosomes, argosomes, dexosomes, ectosomes, exovesicle, oncosome, prominosome, prostasome, tolerosome, microparticle, nanovesicle, blebbing vesicle, budding vesicle, exosome-like vesicle, matrix vesicle, membrane vesicle, shedding vesicle, membrane particle, shedding microvesicle, membrane bleb, epididymosome, promininosome, texosome, or archeosome. However, the present disclosure is not limited thereto. Further, unless otherwise specified in the present disclosure, extracellular vesicle, and exosome may be used interchangeably with microvesicles regardless of sizes thereof.

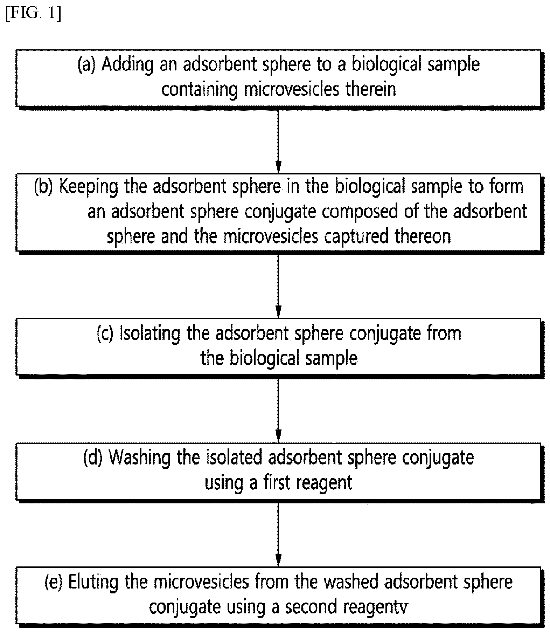

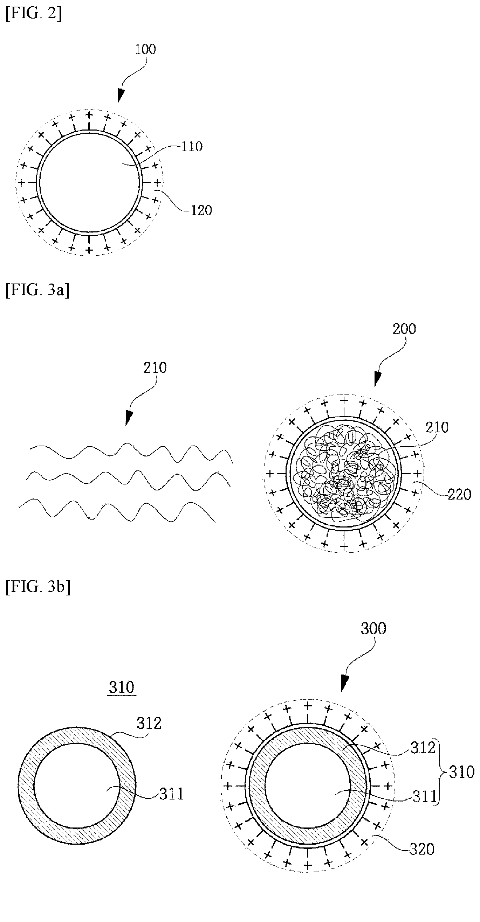

FIG. 1 is a flowchart showing a microvesicles isolation method according to an embodiment of the present disclosure. FIG. 2 is a diagram schematically showing an adsorbent sphere according to an embodiment of the present disclosure. FIG. 3A is a diagram schematically showing an adsorbent sphere according to another embodiment of the present disclosure. FIG. 3B is a diagram schematically showing support according to still another embodiment of the present disclosure.

Referring to FIG. 1, an embodiment of the present disclosure relates to a microvesicles isolation method that isolates the microvesicles contained in the biological sample, the method may include (a) adding an adsorbent sphere to a biological sample containing microvesicles; (b) maintaining the adsorbent sphere in the biological sample to form an adsorbent sphere conjugate composed of the adsorbent sphere and the microvesicles captured by the adsorbent sphere; (c) isolating the adsorbent sphere conjugate from the biological sample; (d) washing the isolated adsorbent sphere conjugate using a first reagent; and (e) eluting the microvesicles from the washed adsorbent sphere conjugate using a second reagent. The adsorbent sphere may include a support and one or more polyvalent cations disposed on a surface of the support.

In the step (a), the adsorbent sphere may be added into the biological sample. The biological sample may include a biological sample, a cell culture medium, a tissue sample, etc. containing microvesicles. Specifically, the biological sample may include at least one of blood, plasma, serum, urine, saliva, cerebrospinal fluid, tears, sweat, feces, ascites, amniotic fluid, semen, milk, cell medium, tissue extract, and cancer tissue.

The step (a) may further include preparing the adsorbent sphere. The adsorbent sphere 100 may include a support 110 and a polyvalent cation 120 coupled to an outer face of the support 110. Specifically, the adsorbent sphere 100 may include the support 110 having a reactive group or a functional group having reactivity on a surface thereof, and the polyvalent cation 120 having a group that chemically reacts with the reactive group or the functional group of the support 110.

The support 110 may include at least one of polyacrylate, polyacrylamide, polymethacrylate, polyethylene glycol, polystyrene vinylbenzene, polystyrene, hydrogel, agarose, ceramic, silica gel, latex, metal particles, glass particles, magnetic particles, metal wires, and metal wires having magnetic nanoparticles coupled thereto.

The polyvalent cation 120 may include at least one of polylysine, polyarginine, polyhistidine, protamine, cationic dextran, cationic dendrimer, cationic polysaccharide, polyamidoamine, polyethyleneimine, polyquaternium, poly-2-dimethylaminoethyl methacrylate (PDMAEMA), poly(2-dimethylaminomethyl styrene) (PDMAMS), poly-1-vinylpyrrolidone (p1-VP), poly diethylamino ethyl acrylate (pDEAEA), poly dimethylaminoethyl acrylate (pDMAEA), and poly diethylaminoethyl methacrylate (pDEAMA). Further, the polyvalent cation may include at least one of lipopolyamines, quaternary ammoniums, guanidine, imidazole, polyaniline, polypyrrol, and chitosan.

Specifically, the support 110 may be present in a form of a polymer having a functional group by itself, or in a form of a polymer having a functional group introduced thereto via plasma treatment, or via surface coating using a polymer having the functional group, or the like.

More specifically, the support 110 may be present in a form of a hydrogel, and a surface area of the adsorbent sphere may increase overall due to a porous nature of the hydrogel. Therefore, the support 110 in the form of the hydrogel may have an increased area in contact with the microvesicles, such that the support 110 may have further improved capturing ability of the microvesicles.

For example, when the support 110 is made of polyacrylate, polyacrylamide, polymethacrylate, polyethylene glycol, etc. and thus has a reactive group or a functional group, the polyacrylate, polyacrylamide, polymethacrylate, polyethylene glycol, etc. may act as the support by itself. Further, when the support itself includes metal particles, glass particles, magnetic particles, etc. and thus does not have a reactive group or functional group, the metal particles, glass particles, magnetic particles, etc. may be subjected to surface treatment or surface coating using a substance having a reactive group or functional group.

The polyvalent cation 120 may chemically bond with the support 110 and may cover at least a portion of the surface of the support 110. The adsorbent sphere 100 has a positive charge due to the polyvalent cation.

Specifically, the support 110 may have a spherical shape. An average diameter of the support 110 may be in a range of 200 nm to 100 µm, and specifically, 500 nm to 3 µm.

In accordance with the present disclosure, a size of the surface area becomes inversely proportional to a square of a bead diameter. Thus, as a size of the support 110 becomes smaller, the efficiency of forming the adsorbent sphere conjugate composed of the adsorbent sphere and the microvesicles captured by the adsorbent sphere increases. However, in order to effectively collect the adsorbent sphere after the isolation of the microvesicles from the adsorbent sphere conjugate, the average diameter of the support may be within the range as described above.

Further, the adsorbent sphere 100 should effectively capture microvesicles among various substances contained in the biological sample. In order to prevent other proteins other than microvesicles contained in the biological sample from adhering to the adsorbent sphere 100 as much as possible, and to effectively capture microvesicles having a predetermined size, the average diameter of the support may be preferably in a range of 500 nm to 3 µm.

In one embodiment, the support 110 may include a bead-type plastic (e.g., polystyrene), or a bead-type metal (e.g., iron (Fe)) having a surface functionalized with a carboxyl group or an amine group. The polyvalent cation 120 may include a cationic polymer.

In another embodiment, the support 110 may be a hyaluronic acid microbead. The adsorbent sphere 100 may include a structure in which the polyvalent cation 120 is bonded to the surface of the hyaluronic acid microbead. When, in this way, the hyaluronic acid microbead is used as the support, a cationic polymer and an aqueous solution of hyaluronic acid are mixed with each other and then a weakly acidic aqueous solution is added to the mixture and the mixture is subjected to electrospinning to prepare the hyaluronic acid microbead (refer to Korean Patent No. 10-1212258).

In another embodiment, the support may include at least one of porous particles, membranes, and meshes. A pore size of the membrane or the mesh may be in a range of 200 nm to 100 µm. Preferably, the pore may have a size in a range of 500 nm to 2 µm. The membrane or the mesh may be made of a plastic material, various metal materials, or materials having magnetic property. However, the present disclosure is not limited thereto.

Alternatively, unevenness may be formed on the surface of the support. Since the unevenness formed on the surface of the support increases the surface area of the support, this may improve the binding force to the polyvalent cation. Further, in the adsorbent sphere including the support and the polyvalent cation disposed on the surface of the support, the pattern of the unevenness formed on the surface of the support may be substantially similarly transferred to the polyvalent cation such that the overall surface area of the adsorbent sphere may also be increased. As a result, an area by which the adsorbent sphere may capture the microvesicles is increased. The microvesicles may be coupled to the polyvalent cation as the surface of the adsorbent sphere via electrostatic attraction, and at the same time, may be fixedly inserted into an inner narrow gap defined by the unevenness of the surface of the adsorbent sphere. In this way, the microvesicles may be captured by the adsorbent sphere. Thus, the microvesicles may be more firmly captured by the adsorbent sphere.

In one embodiment of the present disclosure, the polyvalent cation 120 may include at least one of poly-L-lysine polymer (PLL) having a molecular weight of 150 to 300 kDa, protamine, quaternary ammoniums, chitosan, and polyhistidine. The support 110 may include polystyrene having a carboxyl group.

For example, the adsorbent spheres 100 may be contained in the biological sample at a concentration of 0.5 mg/mL to 20 mg/mL, or 1 mg/mL to 20 mg/mL, or 3 mg/mL to 20 mg/mL, or 5 mg/mL to 20 mg/mL, or 0.5 mg/mL to 18 mg/mL, or 0.5 mg/mL to 15 mg/mL, or 1 mg/mL to 15 mg/mL. Specifically, the adsorbent spheres 100 may be contained in the biological sample at a concentration of 5 mg/mL to 15 mg/mL.

When the adsorbent sphere 100 is excessively contained in the biological sample, a higher content of proteins smaller than the microvesicles may be contained in a void formed between the adsorbent spheres 100 rather than on the surface of the adsorbent sphere 100. Thus, the capturing ability of the microvesicles may be reduced. When a content of the adsorbent spheres 100 is too low, the adsorption efficiency of the microvesicles thereon is low.

Further, the support 110 may be a magnetic bead containing one or more metals selected from the group consisting of iron (Fe), nickel (Ni), cobalt (Co), manganese (Mn), bismuth (Bi) and zinc (Zn) and alloys thereof, or a metal oxide or alloy oxide of a metal selected therefrom.

When the support 110 is the magnetic bead, the polyvalent cation may be coated on the surface of the support. In this regard, the surface of the support 100 may be modified using plasma treatment, electron beam treatment, or the like. For example, the support 100 may be input into a chamber where plasma is generated and then may be treated with plasma having a predetermined energy, such that positive ions, negative ions, electrons, or the like are present on the surface of the support 100, or the functional group or the reaction group may be disposed thereon. Alternatively, when the surface of the support 100 is surface-treated using a stronger energy such as an electron beam, irregularities may be formed on the surface of the support 100. When the surface of the support 100 is plasma-treated, this may improve the binding force to the polyvalent cation. When the unevenness or irregularities are formed on the surface of the support 100 to increase the surface area to be combined with the polyvalent cation, the support 100 may be more effectively combined with the polyvalent cation.

Alternatively, when the support 100 is the magnetic bead, a surface thereof may be modified to have a positive charge (+). Specifically, the surface of the magnetic bead may be coated with silica and then the silica surface may be modified with a positively charged functional group, for example, an amine group such as 3-aminopropyl group, aminomethyl group, 2-aminoethyl group, N-methyl-3-aminopropyl group, N-(2 -aminoethyl)-3-aminopropyl group, N-aminoethyl-3-aminopropyl group or N,N-di(2-aminoethyl)-3-aminopropyl group.

Alternatively, as shown in FIG. 3A, the support 210 may be formed by entangling one or more wires, such as a metal wire or a magnetic wire. In this regard, surface treatment may be performed on a surface of the wire before entangling one or more wires. Alternatively, nano-sized magnetic nanoparticles may be attached to the surface of the wire before entangling one or more wires. After the support 210 is formed by entangling one or more wires into an approximately round shape, the polyvalent cation 220 may be coated on the surface of the support to prepare the adsorbent sphere 200.

Further, alternatively, the support 310 may include a core 311 and a shell 312 provided to surround an outer face of the core 311.

In this regard, when the core 311 is made of at least one of polyacrylate, polyacrylamide, polymethacrylate, polyethylene glycol, polystyrene vinylbenzene, polystyrene, hydrogel, agarose, ceramic, silica gel, and latex, the shell 312 may be made of one or more metals selected from the group consisting of iron (Fe), nickel (Ni), cobalt (Co), manganese (Mn), bismuth (Bi) and zinc (Zn), or alloys thereof, or metal oxides or alloy oxides of metals selected therefrom.

Further, the core 311 may be made of at least one metal selected from the group consisting of iron (Fe), nickel (Ni), cobalt (Co), manganese (Mn), bismuth (Bi), and zinc (Zn) or an alloy thereof or a metal oxide or alloy oxide of a metal selected therefrom. The shell 312 may be made of at least one of polyacrylate, polyacrylamide, polymethacrylate, polyethylene glycol, polystyrene vinylbenzene, polystyrene, hydrogel, agarose, ceramic, silica gel, and latex.

As described above, the core 311 and the shell 312 formed to surround the outer face of the core 311 may be made of different materials. In this regard, at least one of the core 311 or the shell 312 may be made of a material isolated via magnetic isolation. In this regard, the magnetic isolation may be performed by causing an electric current to flow through an electromagnet, or using a magnet.

The material of each of the core 311 and the shell 312 may be variously selected depending on a type of the biological sample, the characteristics of the microvesicles to be captured from the biological sample, or the like.

A polyvalent cation 320 includes at least one of poly-L-lysine polymer (PLL), protamine, quaternary ammoniums, chitosan, and polyhistidine. The polyvalent cation 320 may be provided to cover at least a portion of the shell 312 to form an adsorbent sphere 300.

In the step (b), the adsorbent sphere is maintained in the biological sample to form the adsorbent sphere conjugate in which the adsorbent sphere and the microvesicles are combined to each other. The adsorbent sphere may have an electrically positive (+) charge due to the polyvalent cation, and the microvesicles may have an electrically negative (-) charge. Accordingly, the microvesicles may be coupled to the adsorbent sphere via at least one of electrostatic interaction and intercalation. A zeta potential of the adsorbent sphere conjugate may be in a range of 0.5 mV to 10 mV.

The adsorbent sphere and the microvesicles have (+) and (-) charges, respectively, and thus are combined with each other. In this regard, when the zeta potential of the adsorbent sphere conjugate is lower than 0.5 mV, the microvesicles is not effectively captured by the adsorbent sphere, and a large amount of the microvesicles is lost before the elution with the second reagent as described below, such that the isolation ability of the microvesicles is reduced. To the contrary, when the zeta potential of the adsorbent sphere conjugate exceeds 10 mV, it is difficult to elute the microvesicles with the second reagent. Specifically, the zeta potential of the adsorbent sphere conjugate may be in a range of 1mV to 10mV, or 5mV to 10mV, or 0.5mV to 7mV, or 0.5mV to 5mV.

Further, the adsorbent sphere may have a zeta potential in a range of 10 mV to 30 mV, or 15 mV to 30 mV, or 17 mV to 30 mV, or 20 mV to 30 mV, or 10 mV to 27 mV, or 10 mV to 25 mV, or 12 mV to 25 mV, or 15 mV to 25 mV, or 17 mV to 25 mV, or 20 mV to 25 mV. The adsorbent sphere may have the zeta potential within the above-described range, thereby more effectively capturing the microvesicles in the biological sample.

A size of each of the microvesicles is in an order of nanometers. Thus, it is not easy to isolate the microvesicles from the biological sample using the size exclusion. However, in the microvesicles isolation method according to the present embodiment, the microvesicles are captured by the adsorbent sphere via attraction between positive and negative ions and then are isolated therefrom. In this regard, controlling each of the zeta potential of the adsorbent sphere and the zeta potential of the adsorbent sphere conjugate to be in the above defined range may allow the microvesicles to be effectively isolated from the biological sample.

More specifically, each of the microvesicles captured by the adsorbent sphere has an average diameter of 0.5 nm to 1 µm. The polyvalent cation may include poly-L-lysine polymer (PLL), and the support may include polystyrene having a carboxyl group. More specifically, a size of each of the microvesicles captured by the adsorbent sphere may be in a range of 1 nm to 1 µm, or 10 nm to 1 µm, or 50 nm to 1 µm.

In the step (b), maintaining the adsorbent sphere in the biological sample may include stirring the adsorbent spheres in the biological sample at a temperature of 1 °C to 8 °C, and at 50rpm to 150rpm for 10 minutes to 2 hours.

In the step (b), the stirring temperature may be in a range of 1 °C to 8 °C. When the temperature is lower than 1°C, problems such as freezing of the biological sample may occur. When the temperature exceeds 8°C, the fluidity of substances such as microvesicles and proteins contained in the biological sample is high, which may prevent the microvesicles from being captured by the adsorbent sphere. Specifically, the temperature may be in a range of 2°C to 8°C, or 3°C to 8°C, or 1°C to 7°C, or 1°C to 6°C, or 1°C to 5°C, or 2°C to 7°C, or 3°C to 6°C. More specifically, the temperature may be in a range of 3.5 °C to 4.5 °C. The above temperature range stabilizes proteins acting as impurities other than the microvesicles, thereby inhibiting the fluidity of the proteins, such that the microvesicles may be more effectively captured by the adsorbent sphere.

In the step (b), the stirring may be performed at a stirring speed of 50 rpm to 150 rpm and for a stirring time duration of 10 minutes to 2 hours. When the stirring speed is lower than 50 rpm, the microvesicles are not uniformly adsorbed on the adsorbent sphere and some thereof may be aggregated. When the stirring speed is higher than 150 rpm, the microvesicles may not be stably coupled to the surface of the adsorbent sphere. Specifically, the stirring speed may be in a range of 70rpm to 150rpm, or 100rpm to 150rpm, or 50rpm to 170rpm, or 50rpm to 160rpm, or 70rpm to 100rpm.

The stirring time duration may be in a range of 10 minutes to 2 hours. When the duration is smaller than 10 minutes, it is difficult for the microvesicles to be sufficiently captured by the adsorbent sphere. When the duration exceeds 2 hours, it takes time unnecessarily to increase the microvesicles isolation time. Specifically, the stirring time duration may be in a range of 30 minutes to 2 hours, or 50 minutes to 2 hours, or 1 hour to 2 hours, or 10 minutes to 1.5 hours, or 10 minutes to 1 hour, or 10 minutes to 50 minutes, or 20 minutes to 50 minutes.

A time duration required to form the adsorbent sphere conjugate may be significantly reduced compared to a time duration required in a conventionally used method, for example, an immunoaffinity isolation method.

The step (c) may isolate the adsorbent sphere conjugate from the biological sample. The adsorbent sphere conjugate may have a structure in which the microvesicles is bonded to the surface of the adsorbent sphere.

In the step (c), the adsorbent sphere conjugate is isolated using an isolation member. The isolation member may employ at least one of a capturing filter, magnetic isolation, centrifugal isolation, solubility isolation, and isolation based a particle size.

For example, when the isolation member is a capturing filter, the capturing filter may be a membrane having pores of a size in a range of 50 nm to 1,000 nm, or 100 nm to 500 nm, or 150 nm to 300 nm. When a size of the pores of the capturing filter is within the aforementioned range, the adsorbent sphere conjugate may be isolated from the biological sample using the capturing filter.

The adsorbent sphere conjugate passes through the capturing filter as follows: the biological sample passes through the capturing filter by gravity or negative pressure while the capturing filter is installed inside a reactor, an extraction tube, etc. containing the adsorbent sphere conjugate and the biological sample. Further, the biological sample passes through the capturing filter by gravity or negative pressure while the capturing filter is installed out of the reactor, an extraction tube, etc. containing the adsorbent sphere conjugate and the biological sample such that the capturing filter is adjacent to an outlet of the reactor, the extraction tube, etc.

For example, the capturing filter may be made of a hydrophilic material or a cation exchange resin so that the adsorbent sphere conjugate having an electrical charge (+) is more easily captured by the capturing filter.

When the capturing filter is made of a hydrophilic material, the adsorbent sphere conjugate may be easily caught in the pores and may be physically captured by the filter. The hydrophilic material may include silver metal, polyethersulfone, glass fiber, polycarbonate track etch (PCTE), polyester, mixed cellulose esters (MCE), nylon, cellulose acetate, or a combination thereof.

When the capturing filter is made of a cation exchange resin, the adsorbent sphere conjugate is adsorbed to the surface of the negatively charged capturing filter under an electrical force. The cation exchange resin may include carboxymethyl, methyl sulfonate, a sulphonyl group, or a combination thereof.

Specifically, the capturing filter may have a structure such that the adsorbent sphere conjugate is captured by a cation exchange resin having a negative charge via an electrical force.

In an embodiment of the present disclosure, the microvesicles aggregates may be filtered using a syringe filter having a pore size of about 220 nm.

Further, the isolation member may include magnetic isolation. The support may include a magnetic particle. The adsorbent sphere conjugate may be isolated using a device that causes current to flow in an electromagnet or using a magnet.

Specifically, when using the electromagnet, the adsorbent sphere conjugate is held by electromagnetic waves and thus movement thereof is restricted. Further, when using the magnet, the magnet may be placed at a predetermined position, and then, the adsorbent sphere conjugate may be fixed to the magnet, and then may be isolated from the biological sample. The magnet may be disposed in the inside and/or the outside of the reactor in which the biological sample and the adsorbent sphere are received. In order to prevent the adsorbent sphere from being attached to an inner wall of the reactor, or from settling down to a bottom of the reactor, the magnet may be provided in a form of a rotatable impeller.

In the step (d), the adsorbent sphere conjugate isolated in the step (c) may be washed under a first condition using the first reagent. The first reagent isolates impurities such as substances other than microvesicles captured on the surface of the adsorbent sphere conjugate, for example, proteins having a negative charge, from the adsorbent sphere, thereby improving the purity of the finally isolated microvesicles.

The first reagent includes at least one of chaotropic substance (chaotropic salt), protease, CH₃COO⁻, SO₄²⁻, HCO⁻, SiO⁻, and OH⁻. The first condition may include performing washing one or more times within a pH range of at least one of pH 5.5 to 6.5, or pH 7.5 to 9.5.

Further, a concentration of the first reagent may be in a range of 0.1 to 4.5 M, 0.5 to 4 M, and 1 to 3 M. The first reagent may be provided such that when the first reagent is used a plurality of times, the concentration of the first reagent may gradually decrease.

The chaotropic substance refers to a substance or an ion that destabilizes or destroys a cargo structure of water molecules in an aqueous solution. Due to the addition of the chaotropic substance into the aqueous solution, the entropy of water is increased to affect a force between proteins, a hydrogen bond, Van der Waals force, or hydrophobic bonds such that its molecular structure is destabilized.

The chaotropic substance may include a salt including a guanidinium ion, such as guanidinium isothiocyanate, guanidinium thiocyanate, or guanidinium chloride, or guanidine hydrochloride, n-butanol, ethanol, lithium perchlorate, lithium acetate, magnesium chloride, phenol, 2-propanol, sodium dodecyl sulfate, thiourea, urea, or a combination thereof. However, the present disclosure is not limited thereto.

The protease may include proteinase K, pepsin, trypsin or chymotrypsin.

The first reagent may allow a state in which the microvesicles is attached to the adsorbent sphere in the adsorbent sphere conjugate, and may cause other substances than the microvesicles to be isolated from the adsorbent sphere. The first reagent is provided to maintain the binding force between the microvesicles and the adsorbent sphere, but to isolate other substances than the microvesicles from the adsorbent sphere.

The first condition may include one of pH 5.5 to 6.5, and pH 7.5 to 9.5, and the pH should be within the above-mentioned range, such that the impurities such as proteins may be easily isolated from the adsorbent sphere conjugate, and re-adsorption of the impurities such as the isolated proteins to the adsorbent sphere may be prevented.

Specifically, the first reagent may include guanidium thiocyanate at a concentration of 2M.

Alternatively, the first reagent may not include a chaotropic substance. Further, the first reagent may have an acidic or neutral pH, for example, a pH of 3.0, 3.5, 4.0, 4.5, 5.0. 5.5, 6.0, 6.5, 7.0, 7.5, 8.0, 8.5, or 9.0. The first reagent may be used once or plural times. When the first reagent is used in multiple times, the first reagents respectively used in multiple times may have the same or different pH values within the pH range of one of pH 5.5 to 6.5 and pH 7.5 to 9.5. For example, the pH values of the first reagents respectively used a plurality of times may be gradually decreased or increased.

For example, the pH value of the first reagent as used at a first time may be maintained in a low range to isolate impurities such as the protein from the adsorbent sphere conjugate in a short time. Then, the pH value of the first reagent as used at a second time may be higher than the low range such that the impurities such as the isolated protein may be prevented from being re-adsorbed to the adsorbent sphere. Specifically, the pH value of the first reagent as used at the first time may be acidic in a range of 3 inclusive to 7 exclusive, and the pH of the first reagent as used at the second time may be basic in a range of 7 exclusive to 12. Specifically, the pH value of the first reagent as used at the first time may be in a range of 3 to 6, or 3 to 5, or 3 to 4.5. The pH of the first reagent as used at the second time may be in a range of 7.5 to 12, or 8 to 11, or 9 to 10. Further, the washing may be performed one or more times using the pH value of the first reagent as used at the first time in the range of 3 inclusive to 7 exclusive. Further, the washing may be performed one or more times using the pH value of the first reagent as used at the second time in the range of 7 exclusive to 12.

In one example, the first reagent may be used two or more times. When the first reagent is used two or more times, the pH of the first reagent used at a first time may be in a range of 5.5 to 6.5, and the pH of the first reagent used at a second time, if any, a third time, ... may be in a range of 5.5 to 6.5. Further, alternatively, when the first reagent is used two or more times, the pH of the first reagent used at a first time may be in a range of 5.5 to 6.5, and the pH of the first reagent used at a second time, if any, a third time, ... may be in a range of 7.5 to 9.5.

In the first condition, the temperature may be in range of 3°C to 30°C, or 4°C to 30°C, or 5°C to 30°C, or 10°C to 30°C, or 15°C to 30°C, or 20°C to 30°C, or room temperature.

In the first condition, the time duration may be in a range of 5 seconds to 24 hours. However, the present disclosure is not limited thereto, and the time duration may be variously modified.

In one embodiment of the present disclosure, a 0 to 10 mM NaCl solution of pH 6 or 8.5 may be used as the first reagent. In another embodiment, the first reagent may be used sequentially at pH 6.0, 8.5, and 13.

When the pH of the first reagent changes gradually, the isoelectric point characteristics of the anionic protein aggregated with the microvesicles may be changed such that the impurities such as proteins may be isolated from the adsorbent sphere conjugate and may be washed away. In this regard, the microvesicles have a stronger negative charge than those of most of the proteins. Thus, even when the first reagent is acidic (e.g., pH 4.0), the microvesicles are maintained while not being isolated from the adsorbent sphere conjugate.

The first reagent may include an appropriate salt that may effectively wash the impurities. In one embodiment, the salt may include at least one or more of CH₃COO⁻, SO₄²⁻, HCO⁻, SiO⁻, and OH⁻ at a concentration lower than 50 mM. Further, the first reagent may further include Tris-Cl (pH 8), phosphate, or a combination thereof at a concentration of 10 to 500 mM, and may further include imidazole at a concentration of 10 to 100 mM. However, the present disclosure is not limited thereto. The first reagent may include, without limitation, an additional component that may effectively isolate the impurities such as proteins other than microvesicles from the adsorbent sphere conjugate including an anion exchange resin.

In the step (e), the microvesicles may be eluted from the washed adsorbent sphere conjugate in a second condition and using the second reagent.

The second reagent includes at least one of chaotropic substance (chaotropic salt), protease, CH₃COO⁻, SO₄²⁻, Cl⁻, HCO⁻, SiO⁻, and OH⁻, at a concentration of 0.2 M to 3 M range. Under the second condition, the elution may be performed one or more times at 500 rpm to 2000 rpm and for a time of 1 minute to 60 minutes.

In the step (e), the adsorbent sphere and the microvesicles may be isolated from each other in a state in which the impurities such as proteins have been removed from the adsorbent sphere conjugate. The second reagent may cancel the electrical bonding force between the microvesicles and the adsorbent sphere in the adsorbent sphere conjugate, thereby isolating the microvesicles from the adsorbent sphere.

Conventionally, in order to isolate the microvesicles, an expensive and large-volume device such as a centrifugal isolation device is used. It takes a long time to isolate the microvesicles using the device. Further, it is difficult to isolate the microvesicles at high purity using the device.

On the contrary, in this embodiment, the adsorbent sphere and the appropriately controlled first and second reagents may be used. Specifically, the adsorbent sphere and the microvesicles may be combined with each other into the adsorbent sphere conjugate and then the conjugate may be isolated from the biological sample, and then only the microvesicles may be effectively isolated at the high purity from the adsorbent sphere conjugate. Further, this embodiment does not require a large device such as the centrifugal isolation device, and rather, the method may be performed relatively simply in the outside. The isolation device in accordance with the present disclosure may be used as a diagnostic kit for various purposes without space and time restrictions.

The second reagent and the first reagent may be made of the same substance or different substances. In this regard, the pH values or the concentration values of the second reagent and the first reagent may be different from each other.

Specifically, the first reagent may include a chaotropic substance or protease, while the second reagent may include a chaotropic substance or protease, wherein a concentration of the chaotropic substance or protease in the second reagent may be higher than that of the chaotropic substance or protease in the first reagent.

In one embodiment, when the first reagent is made of a chaotropic substance, the second reagent may be made of a chaotropic substance. When the same chaotropic substance is used for the first and second reagents, the concentration of the chaotropic substance in the first reagent may be lower than the concentration of the chaotropic substance in the second reagent. For example, the concentration of the chaotropic substance in the first reagent may be in a range of 0.1 to 4.5 M, or 0.5 to 4 M, or 1 to 3 M, while the concentration of the chaotropic substance in the second reagent may be in a range of 4.5 M exclusive to 15M, or 4.5 M exclusive to 10M, or 4.5 M exclusive to 8M, or 4.5 M exclusive to 6M. Specifically, the concentration of the chaotropic substance in the first reagent may be in a range of 1.5M to 3M, while the concentration of the chaotropic substance in the second reagent may be in a range of 4M to 7M.

When the first and second reagents are made of the same chaotropic substance, the second reagent may further include guanidinium ions while the first reagent may be free of the guanidinium ions. Further, the second reagent may include the chaotropic substance at a higher concentration than the concentration of the chaotropic substance in the first reagent.

Alternatively, the second reagent may include a substance other than a chaotropic substance. Even when the second reagent has a pH that may change the isoelectric point of the microvesicles, the microvesicles may not be well isolated from the adsorbent sphere. In this regard, the isolation ability of the microvesicles may be improved by adding an separate salt to the second reagent.

The separate salt may include CH₃COO⁻, SO₄²⁻, Cl⁻, HCO⁻, SiO⁻, OH⁻ or a combination thereof at a concentration of 100 mM to 5 M. For example, the second reagent may include NaCl, CaCl₂ or a combination thereof at a concentration of 250 mM to 5 M. Further, the second reagent may further include Tris-Cl (pH 8), phosphate, or a combination thereof at a concentration of 10 to 500 mM. However, the present disclosure is not limited thereto. The second reagent may further contain, without restrictions, an additional ingredient that may effectively elute the microvesicles from the anion exchange resin.

Further, the second reagent may contain a monovalent cation such as NH₄⁺, Na⁺, or K⁺, a divalent cation such as Ca²⁺ or Mg²⁺, or a combination thereof. For example, the salt in the second reagent may be (NH₄)₂SO₄, Na₂SO₄, NaCl, KCl, CH₃COONH₄, or a combination thereof.

In the same manner, the salt may be contained in the first reagent. In this regard, the salt may be contained therein at a concentration in a range of 0.1 M or greater, 0.25 M or greater, 0.5 M or greater, or 0.75 M or greater to 5 M or lower, or 4 M or lower. Specifically, the second reagent may contain 1 M, 2 M, 3 M or 4 M NaCl. In one embodiment, the second reagent may have a pH of 4 to 9.

The second reagent induces ion exchange in which various anions are exchanged between the adsorbent sphere and the microvesicles.

The process from the step (a) to the step (e) has been completed within a short time and results in a high yield and a high purity. In one embodiment, the process from the step (a) to the step (e) may be completed in less than 60 minutes, preferably less than 45 minutes, more preferably, less than 30 minutes, and most preferably, less than 20 minutes.

In another embodiment of the present disclosure, the microvesicles isolation method may further include a first auxiliary isolation for isolating the adsorbent sphere conjugate after washing the adsorbent sphere conjugate with the first reagent in the step (d); and a second auxiliary isolation for isolating the adsorbent sphere after the elution of the microvesicles with the second reagent in the step (e).

When the support is embodied as a bead having a circular cross-section and made of at least one of polyacrylate, polyacrylamide, polymethacrylate, polyethylene glycol, polystyrene vinylbenzene, and polystyrene, the first auxiliary isolation or second auxiliary isolation may be carried out using at least one of a capturing filter having a pore size of 50 nm to 1,000 nm; and porous anion particles having pores of 50 nm or smaller in size.

In the step (d), the impurities such as proteins other than the microvesicles may be washed and isolated with the first reagent. Then, the adsorbent sphere conjugate from which the impurities have been removed may be subjected to the first auxiliary isolation which may be performed using the capturing filter, etc. When the capturing filter is used for the first auxiliary isolation, the adsorbent sphere conjugate may be isolated from the capturing filter by passing a buffer solution having a negative charge through the capturing filter. In one example, the buffer solution may include a solution containing at least one of Ca²⁺, Mg²⁺, Na⁺, K⁺, NH₄⁺ ions, or a combination of two or more thereof.

The second auxiliary isolation may be performed to further remove residual impurities from the microvesicles after the isolation of the microvesicles from the adsorbent sphere. For example, the second auxiliary isolation may be performed to further improve the purity of the microvesicles, such that impurities that could not be removed in the step (d), such as fibrinogen (when the biological sample is, for example, plasma), etc. may be removed therefrom.

The second auxiliary isolation may be performed using porous anion particles. The porous anion particles may adsorb protein impurities (e.g., fibrinogen, etc.) of a size smaller than a pore size thereof thereon and may remove the protein impurities from the microvesicles. In one embodiment, the size of each of the pores of the porous anion particles may be smaller than 50 nm.

When the support is embodied as a magnetic particle, the first auxiliary isolation or second auxiliary isolation may include magnetic isolation causing a current to flow in an electromagnet, or using a magnet.

In another aspect of the present disclosure, the microvesicles isolation method may further include pre-treating the biological sample before the adsorbent sphere is added thereto. The pre-treatment of the biological sample may include filtering the biological sample so that a substance having an average diameter of 0.8 µm or larger is isolated from the biological sample.

When the biological sample is filtered to remove the substance having an average diameter of 0.8 µm or greater therefrom in advance before adding the adsorbent sphere to the biological sample, the adsorbent sphere may more easily capture the microvesicles in the biological sample.

According to another aspect of the present disclosure, a reagent kit for microvesicles isolation that isolates microvesicles from a biological sample may be provided.

The reagent kit for microvesicles isolation that isolates microvesicles contained in the biological sample may comprises: one or more adsorbent spheres having a binding ability to the microvesicles; a first reagent containing at least one of SO₄²⁻, Cl⁻, HCO⁻, SiO⁻, and OH⁻; and a second reagent containing at least one of SO₄²⁻, Cl⁻, HCO⁻, SiO⁻, and OH⁻ at a higher concentration thereof than a concentration thereof in the first reagent, wherein the adsorbent sphere may include a support, and one or more polyvalent cations disposed on a surface of the support.

The reagent kit may further include an isolation member for isolating the adsorbent sphere or the adsorbent sphere having the microvesicle captured thereon. The isolation member may include at least one of a capturing filter, magnetic isolation, centrifugal isolation, solubility-based isolation, and isolation based on a particle size.

The support may be embodied as a bead having a circular cross-section and made of at least one of polyacrylate, polyacrylamide, polymethacrylate, polyethylene glycol, polystyrene vinylbenzene, and polystyrene. In this regard, the polyvalent cation disposed on the surface of the support may include one or more of polylysine, polyarginine, polyhistidine, protamine, cationic dextran, cationic dendrimer, cationic polysaccharide, polyamidoamine, polyethylenimine, polyquaternium, poly-2-dimethylaminoethyl methacrylate (PDMAEMA), poly(2-dimethylaminomethyl styrene) (PDMAMS), poly-1-vinylpyrrolidone (p1-VP), poly diethylaminoethyl acrylate (pDEAEA), poly dimethylaminoethyl acrylate (pDMAEA), and poly diethylaminoethyl methacrylate (pDEAMA). The isolation member may include a capturing filter having pores having a size of 50 nm to 1,000 nm.

Alternatively, the support may include magnetic particles. In this regard, the polyvalent cations may be coated with the surface of the magnetic particle to form the adsorbent sphere. The polyvalent cation may include at least one of polylysine, polyarginine, polyhistidine, protamine, cationic dextran, cationic dendrimer, cationic polysaccharide, polyamidoamine, polyethyleneimine, polyquaternium, poly-2-dimethylaminoethyl methacrylate ( PDMAEMA), poly(2-dimethylaminomethyl styrene) (PDMAMS), poly-1-vinylpyrrolidone (p1-VP), poly diethylaminoethyl acrylate (pDEAEA), poly dimethylaminoethyl acrylate (pDMAEA), or poly diethylaminoethyl methacrylate (pDEAMA). In this regard, the isolation member may include a magnetic isolation causing a current to flow in an electromagnet, or using a magnet.

Further, the reagent kit for microvesicles isolation may further include porous particles that may adsorb, thereon, the impurities such as proteins other than microvesicles contained in the biological sample.

Present Examples and Comparative Examples of the present disclosure are described below. However, the following Examples are only preferred examples of the present disclosure, but the scope of the right of the present disclosure is not limited to the following Examples.

### 1. Isolation experiment using a polyvalent cation-bound polymer support as an adsorbent sphere

### (1) Preparation of Present Example 1, and Comparative Example 1

### Biological sample (Present Example 1, and Comparative Example 1)

This experiment was conducted after obtaining approval from the Institutional Bioethics Research Committee of Korea University Anam Hospital (IRB Project No.: 2016AN0090) and consent from all volunteers for blood samples. Blood samples were taken from the median cubital vein of each volunteer and collected by a 3 mL K2-EDTA vacutainer (Becton Dickinson, USA). After centrifugal isolation of whole blood at 1,900 g for 10 minutes, plasma was isolated by centrifugal isolation at 12,000 g for 15 minutes. Then, the plasma was filtered through a mesh of 800 nm pore size (800 nm filter, Sartorius, Cat no: 91133103) to remove large-sized debris. Each plasma sample was isolated by 1 mL to prepare a biological sample.

### Present Example 1 (extracellular vesicle isolation using PLL-bead, ExoCAS-1)

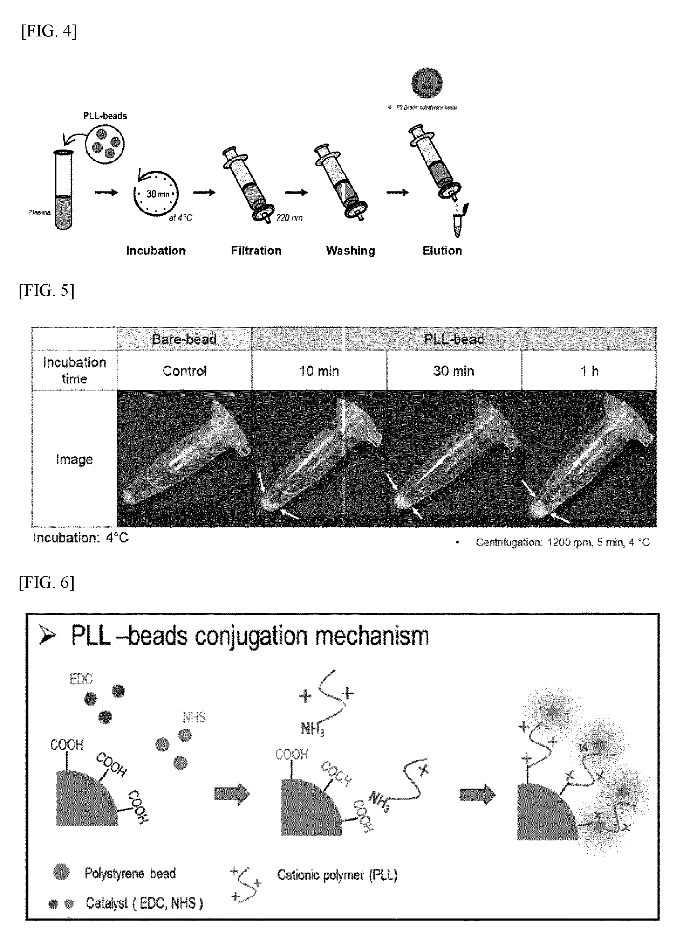

FIG. 4 schematically shows the microvesicles isolation method according to Present Example 1.

Adsorbent spheres were added to the prepared plasma sample (biological sample) to induce aggregation between extracellular vesicles and polymers via the interaction between charges to form a conjugate in which beads and microvesicles were combined to each other, and the conjugate was isolated using a capturing filter.

To prepare the bead having a cationic polymer coupled thereto as the adsorbent sphere, poly-L-lysine (PLL, Sigma-Aldrich, USA) having a molecular weight of 150 to 300 kDa was used as the cationic polymer. After preparing a PLL stock solution of 40 mg/mL concentration, carboxyl group-functionalized beads (1 µm, Carboxyl polystyrene Beads, EPRUI Nanoparticles & Microspheres Co, Shanghai, China) were added to the PLL stock solution and reacted therewith for 6 hours. Thereafter, centrifugal isolation was performed to isolate a solid including the beads, which in turn was washed with phosphate buffered saline (PBS) to prepare the bead having the cationic polymer coupled thereto. In the same manner, each of 5 mg/mL, 20 mg/mL, 40 mg/mL, and 100 mg/mL of PLL stock solutions was used, and the reaction times were set to 3 hours, 6 hours, and 12 hours. Thus, the cationic polymer-conjugated beads (PLL-beads) were prepared. The binding performances thereof to extracellular vesicles were compared with each other.

Based on a result of analyzing the binding performance based on the fact that a volume increases when the extracellular vesicles are bound to the PLL-bead, it was identified that the maximum performance was obtained in the 100 mg/mL PLL stock solution, but was substantially equivalent to that of the 40 mg/mL PLL stock solution. Further, when the reaction time was 6 hours, the maximum performance was obtained. When the reaction time was 12 hours, the binding performance decreased slightly. Therefore, it was identified that the optimal condition in terms of economy and efficiency was the 6 hours reaction time and the concentration of 40 mg/mL. The prepared cationic polymer-bound beads were adjusted such that the final bead concentration in each plasma sample was 5 mg/mL.

The plasma containing the cationic beads was incubated at 4°C for 10 minutes. After the incubation, the mixture was filtered using a syringe filter (220 nm filter, Millex, R8EA69979) having a pore of 220 nm in size. After the filtration, large sized bead particles were left on the filter.

To wash impurities including plasma proteins bound to the cationic polymer-bound bead, we caused the first reagents at pH 4.0, 8.5, and 13 (a concentration each thereof being 800 µL) to sequentially flow on the surface of the bead placed on the filter. The pH value of the first reagent was adjusted using acetic acid (Sigma Aldrich, Cat no: 695092-100 mL) and sodium hydroxide (Sigma Aldrich, Cat no: S8045-500G). The first reagent further contains a low concentration (10 to 50 mM) NaCl, 10 to 50 mM CaCl₂ solution (pH 7.4), 10 to 500 mM Tris-cl (pH 8), 10 to 500 mM phosphate, or 10 to100 mM imidazole, thereby increasing the washing performance of the protein.

In order to obtain an extracellular vesicle bound to the cationic polymer-bound bead, 200 µL of NaCl aqueous solution 3M (Sodium chloride, Sigma Aldrich, Cat no: S7653-250G) was used as the second reagent. We caused the second reagent to flow on the bead placed on the mesh. To identify the obtained extracellular vesicle, NTA was performed to measure the size and particle concentration of the extracellular vesicle, and the concentration of the protein contained in the second reagent was analyzed via BCA test. Further, SEM images (Quanta 250 FEG; FEI, USA) were photographed and analyzed.

### Comparative Example 1 (UC, extracellular vesicle isolation by ultra-centrifugation isolation)

The ultracentrifugation (UC) is a standard method that has been traditionally and widely used for the isolation of extracellular vesicles. Although the isolation protocol of the UC is simple, the UC is time-consuming (average more than 6 hours) and is labor-intensive. The plasma and phosphate buffered saline (PBS) were mixed with each other in a volume ratio of 1:1, and the mixture was centrifugally isolated to remove residual cell components (4°C, 12,000 g, 30 min). The supernatant was transferred and the centrifugal isolation was repeated once under the same conditions. The supernatant was filtered using a syringe filter having a 200 µm pore size (Merck Millipore, USA), followed by ultra-centrifugation at 120,000 g and 4°C for 2 hours (CP100WX; Hitachi, Japan). After aspirating the supernatant, pellets settling down on the bottom were carefully washed with PBS at 120,000 g and 4°C for 1 hour, and then resuspended in 50 µL of phosphate buffered saline (PBS).

### (2) Experiment of Present Example 1 and Comparative Example 1

### Experimental Example 1 (adsorbent sphere conjugate formation)

After the PLL-beads as a bead to which the cationic polymer is bound, and bare-beads as a bead to which the cationic polymer is not bound were added to the plasma at the same amount, incubation was performed thereon for various time duration at 4°C, and the combination between the beads and extracellular vesicles (adsorbent sphere conjugate) in the form of pellets was identified.

### Experimental Example 2 (Evaluation of first reagent)

As the first reagent, a mixture of guanidinium thiocyanate (GuTc 2M) as a chaotropic substance, acetic acid (Sigma Aldrich, Cat no: 695092-100mL), and sodium hydroxide (Sigma Aldrich, Cat no: S8045) -500G) was used. The pH thereof was controlled to to 4.0 and 8.5 (800 µL). Further, NaCl (Sodium chloride, Sigma Aldrich, Cat no: S7653-250G) was used as a salt. After the bead to which the cationic polymer was bound was washed using the first reagent, the concentrations of proteins and particles contained in the resultant first reagent were identified. In this regard, pH 4 was obtained via mixture of acetic acid and sodium hydroxide at 29 mM and 6.1 mM, respectively. pH 6 was obtained via mixture of acetic acid and sodium hydroxide 29 mM and 29 mM, respectively. pH 8.5 was obtained via mixture of acetic acid and sodium hydroxide at 29 mM and 32.3 mM, respectively. pH 11 was obtained via mixture of acetic acid and sodium hydroxide at 29 mM and 34 mM of, respectively. pH 13 was obtained via mixture of acetic acid and sodium hydroxide at 29 mM and 35 mM, respectively.

### Experimental Example 3 (Second reagent evaluation)

Experimental Example 3 was performed in the same manner as in the Experimental Example 2 except that in the Experimental Example 3, the first reagent had a pH of 4 and was free of salt, and the second reagent had pH 7, and the concentration of NaCl salt therein was set 1M, 2M, 3M, 4M and 5M. The PLL-bead concentration in the second reagent was identified.

### Experimental Example 4 (Evaluation of protein and purity in second reagent based on first reagent)

A solution with a pH of 4, 8.5, or 13 free of the chaotropic salt was used as the first reagent, and the proteins that are impurities were isolated from the PLL-bead using the first reagent. NaCl 3M, pH 7 was used as the second reagent. In this case, proteins and microvesicles in the second reagent were evaluated. In this regard, a purity ratio refers to a concentration of extracellular vesicles (particles/mL) to the protein concentration (µg/mL).

### Experimental Example 5 (evaluation of isolation method)

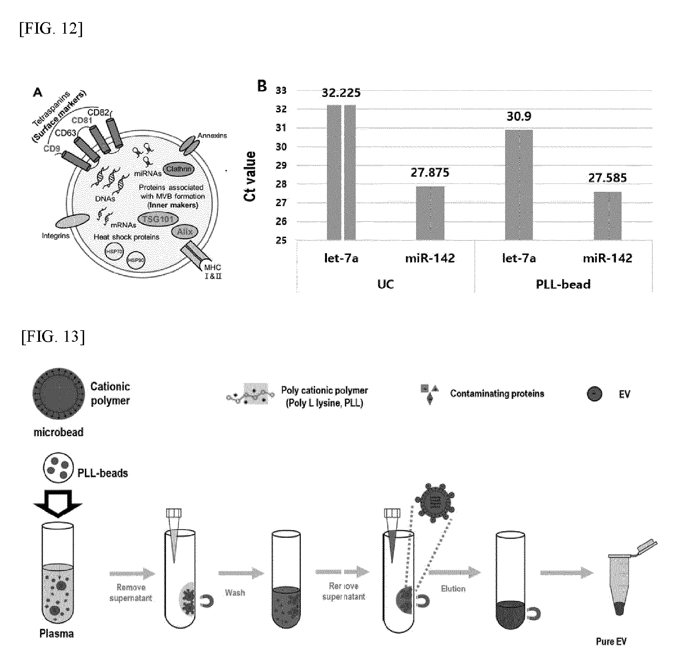

Protein markers of extracellular vesicles isolated using Present Example 1, and Comparative Example 1 to Comparative Example 3 were measured by Western blotting. It is known that the exosome contains protein markers such as Alix (ALG-2-interacting protein X), TSG101 (tumor susceptibility gene 101 protein), HSP70 (heat shock protein), tetraspanins CD63, CD81, and CD9. Accordingly, characteristics of the isolated extracellular vesicles were analyzed using four reference protein markers (ALIX, TSG101: inner protein markers; CD9, and CD81: surface protein markers (A in FIG. 12).

Briefly describing the western blotting process, protein samples were first isolated by gel electrophoresis using SDS-PAGE Mini-PROTEAN^{®} TGX^{™} Precast Gel (456-1035; Bio-rad, USA), and were subjected to immunoblotting using rabbit polyclonal antibody (1:2,000 dilution) anti-CD9 (ab92726), anti-CD81 (ab109201), anti-ALIX (ab186429), anti-TSG101 ( ab125011), and goat anti-rabbit IgG H&L(HRP) (ab205718) (Abcam, Cambridge, UK). The protein band were analyzed using enhanced chemiluminescence (ECL) solution and ChemiDoc^{™} XRS + System (Bio-Rad, USA). As a result of Western blotting of the marker, the total level of the protein marker may be measured from the intensity of the protein band.

Briefly describing the RNA extraction and quantification process, RNA was isolated using the seraMir Exosome RNA purification kit (RA806A-1, SBI, USA). All isolation procedures were performed according to the producer protocol. To quantify EV miRNA markers, reverse transcription was performed on RNA eluate using TaqMan MicroRNA RT Kit (4366596, Life Technologies, USA) and TaqMan Micro RNA Assay (4427975, Life Technologies, USA). In this process, TaqMan Universal Master Mix II, no UNG (4440040, Life Technologies, USA) and miRNA assays hsa-let-7a-5p, ID 000377, and hsa-miR-142-3p, ID 000464 were used. Further experiments were performed on the RNA eluate using an Agilent Eukaryote Total RNA Pico chip on an Agilent 2100 bioanalyzer (Agilent Technologies, USA).

### (3) Evaluation of Present Example 1 and Comparative Example 1

The evaluation results on Present Example 1 and Comparative Example 1 are described below.

FIG. 5 shows a result of identifying the isolation ability of extracellular vesicles using the PLL-bead according to Present Example 1 and the bare-bead as a bead to which a cationic polymer is not bound.

Referring to FIG. 5, the effect of incubation at 4 °C of 1 ml of plasma sample using each of the bare-bead and the PLL-bead was identified. The bare-beads were agglomerated on the bottom of the tube during incubation for 2 hours. The volume of the bare-beads did not change by increasing the incubation time up to 2 hours. To the contrary, it was identified that the volume of the PLL-beads in a form of the pellets gradually increased as the incubation time was increased to 10 minutes, 30 minutes, and 1 hour. That is, it is identified that the bare-bead exists only as a bead itself and cannot capture extracellular vesicles thereon, whereas the PLL-bead binds to the extracellular vesicles contained in the plasma, and thus, as the incubation time increases, an amount of the extracellular vesicles coupled thereto is increased.

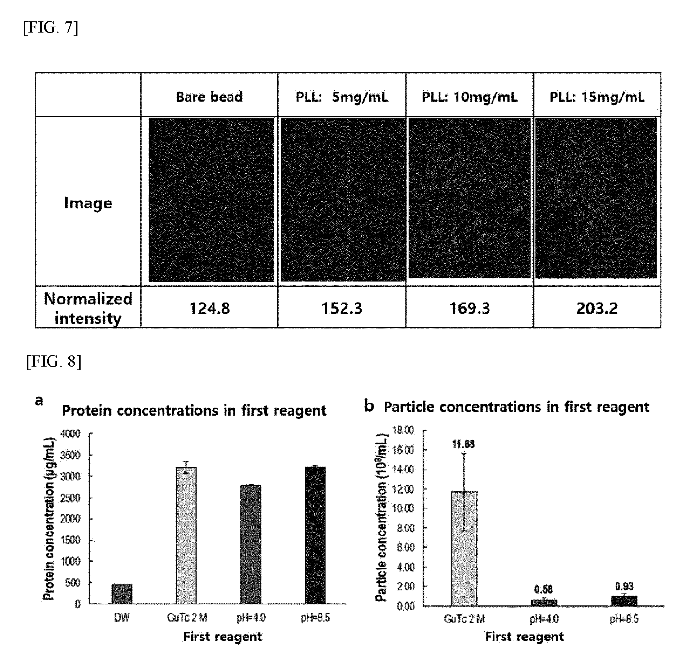

FIG. 6 is a diagram schematically showing how the PLL-bead of Present Example 1 was prepared, and FIG. 7 shows a fluorescent image of the PLL-bead as prepared in a manner according to FIG. 6 while a PLL concentration varies. In this regard, 5 mg of polystyrene was used as the bare-bead, a molecular weight 150 to 300 Kda Poly-L-Lysine was used as the PLL, and an antibody (lgG [Alexa Flour]) was used for the fluorescence image.

Referring to FIG. 6, since the bare-bead includes a functional group of -COOH on a surface thereof, and the PLL has -NH₃ at an end thereof, -COOH and -NH₃ may react with and bind to each other. In this regard, EDC or NHS as catalyst may be further contained to cause the reaction to be carried out more efficiently.

Based on a result of identifying the fluorescence image in FIG. 7, it may be identified that the normalized intensity gradually increases as the concentration of the PLL stock solution is increased to 5 mg/mL, 10 mg/mL, and 15 mg/mL. In other words, it was identified that PLL was effectively attached to the surface of the bare-bead (polystyrene), and this attachment was dependent on the concentration of PLL. That is, it was identified that the PLL-bead according to Present Example 1 was effectively combined with the microvesicles contained in the biological sample.

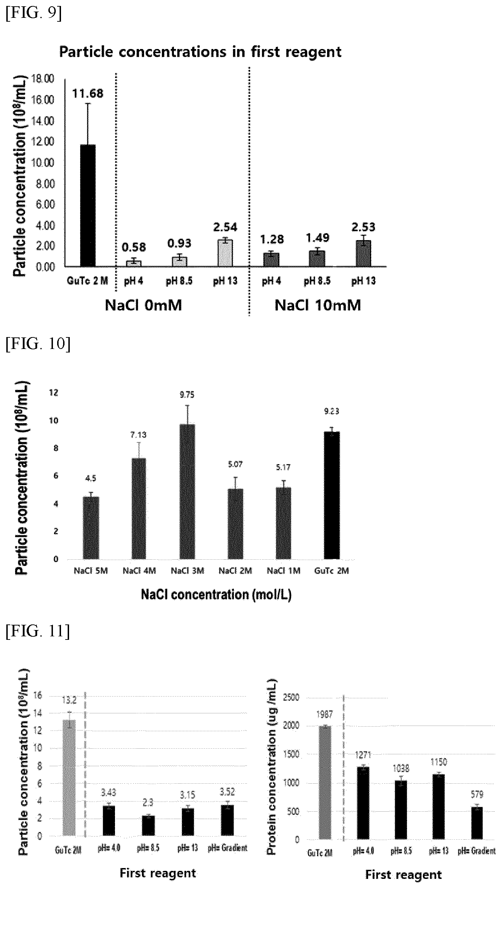

FIG. 8 shows the results of identifying performances based on various first reagents using Present Example 1. FIG. 9 shows the results of identifying the performance based on the first reagent to which the salt was added, using Present Example 1.

In FIG. 8 and FIG. 9, the protein concentration was measured using the BCA test, and the particle concentration was measured using nanoparticle tracking analysis; NTA).

In FIG. 8 and FIG. 9, the concentration of the protein contained in the first reagent is related to the result of isolating the protein as the impurities partially captured onto the bead to which the cationic polymer is bound, and the concentration of the particle is related to the bead to which the cationic polymer is bound.

Referring to a of FIG. 8, it was identified that and all of the first reagents of GuTc, pH 4 and pH 8.5 exhibited higher concentrations of impurities than that in deionized water (DW). That is, it was identified that GuTc, pH 4, and pH 8.5 effectively isolated the impurities attached to the PLL-bead. Further, it was identified that the abilities of GuTc, pH 4, and pH 8.5 to wash and remove the impurities of the PLL-bead were similar to each other.

Referring to b of FIG. 8, it was identified that the protein washing ability of each of the pH of 4 and 8.5 was in a range of 85 to 90% of that of GuTC, and the particle concentration using the pH 4 or 8.5 was superior to that using GuTc by 12.5 to 20.1 times.

Referring to FIG. 9, it may be identified that the amount of loss of the PLL-beads in the first sample is as follows: GuTc < pH 4 or pH 8.5 (containing NaCl salt) < pH 4 or pH 8.5 (free of NaCl salt). Further, a tendency of the loss based on pH occurs. As the pH increases, the amount of the loss of PLL-bead increases. However, it is identified that at pH 13, the loss result is independent of whether the salt was contained. This is because the salt NaCl acts as a base, and pH 13 is a strong base, and the effect at pH 13 is smaller than that when the pH is low.

FIG. 10 shows results of identifying the performances based on various second reagents using Present Example 1.

Referring to FIG. 10, as the particle concentration contained in the second reagent is lower, the microvesicles are isolated while not being lost into the second reagent. In this regard, it was identified that the second reagent was superior to GuTC 2M used as a reference substance at pH 7, and 1M NaCl was the most excellent.

FIG. 11 is a graph showing a purity related to the particles and proteins contained in the second reagent, based on the type of the first reagent.

FIG. 11 shows the particle concentration contained in the second reagent (extracellular vesicle concentration contained in the second reagent) and the protein concentration contained in the second reagent (protein concentration acting as impurities contained in the second reagent) when all of types of the second reagents as used are the same, but, in order to wash the proteins that are impurities before using the second reagent, different types of the first reagents: GuTc 2M, pH 4.0, pH 8.5, pH 13, pH gradient are used at the same volume and the washing is performed once. In this regard, the pH gradient refers to gradually increasing the pH to pH 4.0, pH 8.5, and pH 13.

Referring to FIG. 11, it was identified that all of pH 4.0, pH 8.5, pH 13 and pH gradient exhibited better performance than that of the reference GuTc 2M.

It was identified that when the pH was changed using acetic acid and sodium hydroxide, the amount of protein as detected was lower compared to that using GuTc 2M. In particular, when a plurality of first samples having different pH were used (pH gradient), the smallest amount of protein detected was observed.

It may be identified that the results according to the different types of the first reagents are different from each other while the same second reagent was used. That is, it was identified that even when the second reagent was the same, impurities such as the proteins should be effectively removed in the previous step.

It was identified that the amount of extracellular vesicle contained in the second reagent exhibited the highest purity when a plurality of first reagents having different pH concentrations were used while sequentially increasing the pH. That is, it may be identified that the purity ratio defined as the value obtained by dividing the number of particles by the protein concentration has the highest value in the pH gradient case when the pH of the first reagent is sequentially increased to the pH of 4, 8.5, or 13.

FIG. 12 shows the results of evaluating the conventional centrifugal isolation method and the isolation method according to Present Example 1 of the present disclosure.

In these methods, total RNA inside the microvesicles extracted according to each of the centrifugal isolation method (Comparative Example 1; UC), and the PLL-bead (Present Example 1; PLL-bead) was extracted, and RT-PCR was performed thereon using primers hsa-let-7a-5p and hsa-miR-142-3p, which are extracellular vesicle markers. As a result, it was identified that the amount of RNA as extracted was as follows: PLL-bead (Present Example 1) > centrifugal isolation method (Comparative Example 1). Present Example 1 using the adsorbent sphere and the first and second reagents exhibited the best RNA extraction amount (see B in FIG. 12 below).

### 2. Isolation experiment using magnetic support having polyvalent cation coupled thereto as adsorbent sphere

### (1) Preparation of Present Example 2, Present Example 3, and Comparative Example 2 to Comparative Example 4

### Biological sample (Present Example 2, Present Example 3, and Comparative Example 2 to Comparative Example 4)

Plasma samples used in the following Present Examples and Comparative Examples were purchased from Zen-Bio (Research Triangle, NC, USA). Large-sized samples were removed from the plasma samples using a mesh filter (800 nm pore size mesh, Sartorius Minisart ^{™} NML, catalog number 16592), and a volume of each sample was 1 mL.

### Present Example 2 (extracellular vesicle isolation using PLL-magnetic bead, exoCAS-2)

FIG. 13 is a diagram schematically showing the microvesicles isolation method according to Present Example 2.

After preparing the adsorbent sphere, the adsorbent sphere was added to the prepared plasma sample (biological sample) to induce aggregation between extracellular vesicles and polymers via the interaction between charges to form a conjugate in which the adsorbent spheres and the microvesicles are coupled to each other, and then the conjugate was isolated using a magnetic force.

The PLL-magnetic beads as the adsorbent spheres prepared by reacting 40 mg/ml of poly-L-lysine (PLL, Sigma-Aldrich, St. Louis, MO, USA) polymer and 5 mg of spherical magnetic beads (magnetic beads with a diameter of 1 µm) with each other were prepared and then were added to the plasma sample. PLL has polyvalent cationic properties, and accordingly, the PLL-magnetic beads are coupled with negatively charged extracellular vesicles via electrostatic attraction. In this regard, the biological sample to which the PLL-bead was added was incubated for 30 minutes at 4°C in a rocking platform mixer at 90rpm. During the incubation process, the PLL-magnetic beads exist in a flowing state in the biological sample and are mixed with the sample at a low speed such that excellent bonding between the PLL-magnetic bead and the extracellular vesicle (exosome) is achieved. Thus, hundreds of nanoscale extracellular vesicles were captured on the surface of the PLL-magnetic beads. Then, after holding the mixture for 5 minutes, the magnet was displaced toward the tube containing the biological sample to which the PLL-beads were added, such that the PLL-magnetic beads onto which the extracellular vesicle was captured were isolated. In this regard, the isolation time was as short as 2 minutes, and then the supernatant was isolated using a pipette.

The PLL-magnetic bead onto which the extracellular vesicle was captured was carefully washed using the first reagent (2 mL) of a pH of 6 to remove the non-target protein, and then the PLL-magnetic beads onto which the extracellular vesicle was captured were resuspended in the first reagent. Using a magnet, the PLL-magnetic beads onto which the extracellular vesicles were captured were aggregated and isolated, and in this process, proteins acting as impurities were washed and removed. The PLL-magnetic beads onto which the extracellular vesicles were captured were isolated, and then, the isolated PLL-magnetic beads onto which the extracellular vesicles were captured were added to the second reagent containing a salt of 1M NaCl at pH 7.0 (200 µL), and then the mixture was vortexed at 1000 rpm for 5 minutes. In the vortexing process , the PLL-magnetic bead and the extracellular vesicle were eluted from each other, and the magnet was kept near the tube for 2 minutes, and the PLL-magnetic beads were collected and isolated. The supernatant containing pure extracellular vesicles was collected using a pipette. This entire process was completed in 40 minutes, and molecular analysis and physical property analysis thereon were performed using nanosite (NTA).

### Present Example 3 (extracellular vesicle isolation using protamine-magnetic bead)

Protamine-magnetic beads as the adsorbent spheres were prepared in the same manner as in Present Example 3 except that protamine salt (#P4505, Sigma-Aldrich, St. Louis, MO, USA) of 80 µg was used in place of poly-L-lysine (PLL, Sigma-Aldrich, St. Louis, MO, USA) polymer of 40 mg/ml. We added the prepared adsorbent sphere to the prepared plasma sample (biological sample) to induce aggregation between extracellular vesicles and polymers via interaction between charges to form a conjugate in which the adsorbent spheres and microvesicles were coupled to each other, and then the conjugate was isolated from the sample using a magnetic field.

### Comparative Example 2 (extracellular vesicle isolation using ultra-centrifugation isolation (UC))

The ultracentrifugation (UC) is the most standard method (gold standard method) used to isolate extracellular vesicles. However, it takes about 6 hours or more to isolate extracellular vesicles using the UC. Further, UC requires a lot of labor. To use the ultra-centrifugation isolation, the plasma sample was mixed with phosphate buffered saline (PBS) in a volume ratio of 1:1, and the mixture was centrifugally isolated at 4°C and 12,000 g for 30 minutes to remove residual cell components therefrom. The supernatant was isolated, and centrifugal isolation was repeated thereon under the same conditions. The isolated supernatant was filtered using a 220 µm pore syringe filter (Merck Millipore, Burlington, MA, USA), and the filtered supernatant was subjected to centrifugal isolation using a high-speed centrifugal separator (CP100WX; Hitachi, Tokyo, Japan) at 4°C and 12,000 g for 2 hours. After the supernatant was absorbed and isolated, the remaining pellets were resuspended, washed with phosphate buffered saline (PBS) at 120,000 g and 4°C for 1 hour, and resuspended in 50 µL of PBS.

### Comparative Example 3 (EQ, extracellular vesicle isolation 1 using commercial product)

To isolate extracellular vesicles, a commercially available ExoQuick^{™} exosome precipitation solution (EXOQ5A-1; System Biosciences, Palo Alto, CA, USA) was used. Experimental procedures were carried out in the included manual therein. To use ExoQuick^{™}, the plasma sample was mixed with ExoQuick solution, which is a PEG-based solution, and then the mixture was incubated at 4°C for 30 minutes. After centrifugal isolation of the incubated mixture at 1500 g for 30 minutes, the supernatant was removed, and the remaining pellets were placed in a tube. After performing the second centrifugal isolation thereon at 1500 g for 5 minutes, all remaining ExoQuick solution was removed, and then, the resulting pellets were resuspended in 200 µL of PBS.

### Comparative Example 4 (exoEasy, extracellular vesicle isolation 2 using commercial product)

As described in the manual included in the kit, plasma exosomes were extracted using the exoEasyTM Maxi Kit (Qiagen, Valencia, CA, USA). After filtration thereon to remove particles having a diameter of 0.8 µm or larger, the same volume of XBP buffer solution was added thereto and mixed therewith, and the suspension was transferred to an exoEasy spin column and centrifugal isolation was performed thereon at 500 g for 1 minute. After removing a flow-through, 10 mL of XWP buffer solution was added thereto, and centrifugal isolation was performed on the suspension at 5000 g for 5 minutes. After removing a flow-through, and transferring the spin column to a new tube, 100 µL of XE buffer solution was added thereto to prepare a mixture. The mixture was centrifugally isolated at 5000 g for 5 minutes, and then a flow-through was collected using exosome resuspension solution B (exo-B).

### (2) Experiments on Present Example 2, Present Example 3, and Comparative Example 2 to Comparative Example 4

### Experimental Example 6 (PLL-magnetic bead preparation and incubation conditions)

5 mg of a spherical magnetic beads with an average diameter of 1 µm reacted with PLL at varying the concentrations of 40 mg/ml, 200 mg/ml, and 600 mg/ml to prepare the PLL-magnetic beads and then the extracellular vesicle isolation ability thereof was evaluated. The PLL-magnetic bead with the best performance as prepared by reacting 40 mg/ml of PLL with 5 mg of a spherical magnetic bead with an average diameter of 1 µm was added into the plasma sample. Then, a performance thereof was evaluated based on an incubation time duration, and the incubation temperature.

### Experimental Example 7 (Evaluation of Zeta Potential)

The zeta potential of extracellular vesicles isolated using each of Present Example 2 (PLL-magnetic bead) and Comparative Example 2 (UC) were measured using a zeta potential analyzer (Zetasizer Pro; Malvern Panalytical, Malvern, UK), and the zeta potential of the PLL-magnetic bead (adsorbent sphere) in a micro size of Present Example 2 was measured using the zeta potential analyzer. The pellet-clustered PLL beads (5 mg/mL) prepared in 1 mL plasma were resuspended in 10 µL NaCl solution (1M) (it is difficult to resuspend the pellet-clustered PLL beads in the deionized water (DW)). 990 µL of deionized water was added thereto.

### Experimental Example 8 (Cryo-TEM (Cryo-transmission electron microscopy) evaluation)

The extracellular vesicles isolated using each of Present Example 2 (PLL-magnetic bead) and Comparative Example 2 (UC) were transferred to a 20 nm mesh grid, and then were cultured in a frozen manner (-196°C) for 2 hours using Vitrobot^{™} (FEI, Hillsboro, OR, USA)). After the sample was prepared, extracellular vesicles were identified using a transmission electron microscope (TEM, Tecnai G2-F20, FEI, Hillsboro, OR, USA).

### Experimental Example 9 (Scanning Electron Microscopy (SEM) evaluation)

Extracellular vesicles isolated using each of Present Example 2 (PLL-magnetic bead) and Comparative Example 2 (UC) were filtered using an anodic aluminum oxide membrane (AAO membrane) mounted on a gasket. After the filtration, the AAO membrane was incubated in glutaraldehyde solution (Sigma-Aldrich, St. Louis, MO, USA) for 30 mins. Thereafter, the AAO membrane was sequentially washed with each of 25%, 50%, 75%, 90%, 100% ethanol and incubated overnight at 37°C in a drying oven. After coating the AAO membrane with Pt, SEM (Quanta 250 FEG; FEI, Hillsboro, OR, USA) was used to identify extracellular vesicles and cluster substances on the AAO membrane.

### Experimental Example 10 (Nanoparticle Tracking Analysis (NTA) evaluation)

1mL of of the extracellular vesicle solution as obtained via each of Present Example 2 (PLL-magnetic bead), Present Example 3 (protamine-magnetic bead), Comparative Example 2 (UC), Comparative Example 3 (ExoQuick), and Comparative Example 4 (exoEasy) was isolated for NTA analysis. The sample diluted with PBS was placed in an assembled sample chamber of a NanoSight LM10 system (Malvern Panalytical, Worcestershire, UK) and the microparticles were focused using the fingerprint area as a reference. Extracellular vesicle images were recorded, and a mean particle size and the concentration thereof were measured.

### Experimental Example 11 (Western Blot Analysis Evaluation)

The proteins isolated from exosomes suspended in 200 µL of an elution buffer solution (exosomes isolated by the Experimental Example as described above) were heated in Laemmli buffer solution (Bio-Rad, Hercules, CA, USA) containing 2-mercaptoethanol (Sigma-Aldrich, St. Louis, MO) at 95 °C for 10 mins and thus were modified. The proteins were isolated in an electrophoresis manner using SDS-PAGE Mini-PROTEAN TGX^{™} Precast Gels (456-1035; BioRad). Then, the protein was subjected to immunoblotting using rabbit polyclonal antibody (1:2000 dilution), anti-TSG101 (ab125011), anti-CD81 (ab109201), anti-ALIX (ab186429), anti-CD9 (ab92726), and goat anti-rabbit IgG H&L ( HRP) (ab205718) (Abcam, Cambridge, UK). Protein bands were identified using an enhanced chemiluminescence reagent and ChemiDoc^{™} XRS + System (Bio-Rad).

### Experimental Example 12 (Evaluation of Protein Content)

Pierce^{™} BCA Protein Assay Kit (#23225; Thermo Scientific, Waltham, MA, USA) was used for evaluation of the purity. A standard curve (standard range 0 to 200 µg/mL) was obtained by consecutively diluting bovine serum albumin and working reagents at 9 levels. Three replicates were performed on all samples and standard points. Each 100 µL sample was mixed with 2.0 mL working reagent and incubated at 37°C for 30 minutes. After cooling the mixture to room temperature, we measured a difference between the average absorbance and the absorbance value of the replicate blank standard at 562 nm using a spectrometer (DS-11; Denovix, Wilmington, DE, USA), and then, the difference in the absorbance was converted to µg/mL based on the standard curve. When the protein concentration based on the standard curve exceeds the upper limit of the standard curve (2000 µg/mL), the sample was diluted such that the concentration of the sample became a value within the standard range, and the final sample concentration value was corrected based on the dilution factor.

### Experimental Example 13 (RNA analysis evaluation)

RNA was isolated using a SeraMir Exosome RNA purification kit (RA806A-1, SBI, Palo Alto, CA, USA). All isolation methods were performed according to the manufacturing company's manual. To quantify extracellular vesicle miRNA markers, RNA elution solution was subjected to reverse transcription using TaqMan MicroRNA RT Kit (4366596, Life Technologies, Carlsbad, CA, USA) and TaqMan Micro RNA Assay (4427975, Life Technologies, Carlsbad, CA, USA). TaqMan Universal Master Mix II, no UNG (4440040, Life Technologies, Carlsbad, CA, USA) was used together with miRNAs hsa-let-7a-5p, ID 000377 and hsa-miR-142-3p, ID 000464. An additional experiment was performed on RNA elution using a bioanalysis device (Agilent Eukaryote Total RNA Pico chip on an Agilent 2100 bioanalyzer, Agilent Technologies, Santa Clara, CA, USA).

### Experimental Example 14 (Evaluation of RNA analysis using microarray)

The miRNA expression profile was analyzed using a microarray (Gen-Chip miRNA4.0 array, Affymetrix Inc., Santa Clara, CA, USA). Total RNA (130ng) including tissue miRNA was labeled with biotin using FlashTag. The sample labeled using the TM Biotin HSR RNA labeling kit (Affymetrix) was placed in the Affymetrix miRNA microarray using the GeneChip Hybridization Oven according to the manual of each of the manufacturing companies. The labeled RNA was heated at 99°C for 5 minutes, and then was heated at 45°C for 5 minutes. RNA-array hybridization was performed while performing stirring at 60 rpm at 48 °C for 16 hours, using an Affymetrix^{®} 450 Fluidics station. The chips were washed and stained using a Genechip Fluids Station 450 (Affymetrix), and each chip was scanned using an Affymetrix GCS 3000 scanner (Affymetrix). A value of each signal was identified using Affymetrix^{™} GeneChip^{™} Command Console software (Affymetrix Inc., Santa Clara, CA, USA).

### (3) Evaluation of Present Example 2, Present Example 3, and Comparative Example 2 to Comparative Example 4

The evaluation results on Present Example 2 and Comparative Example 2 to Comparative Example 4 are described below.

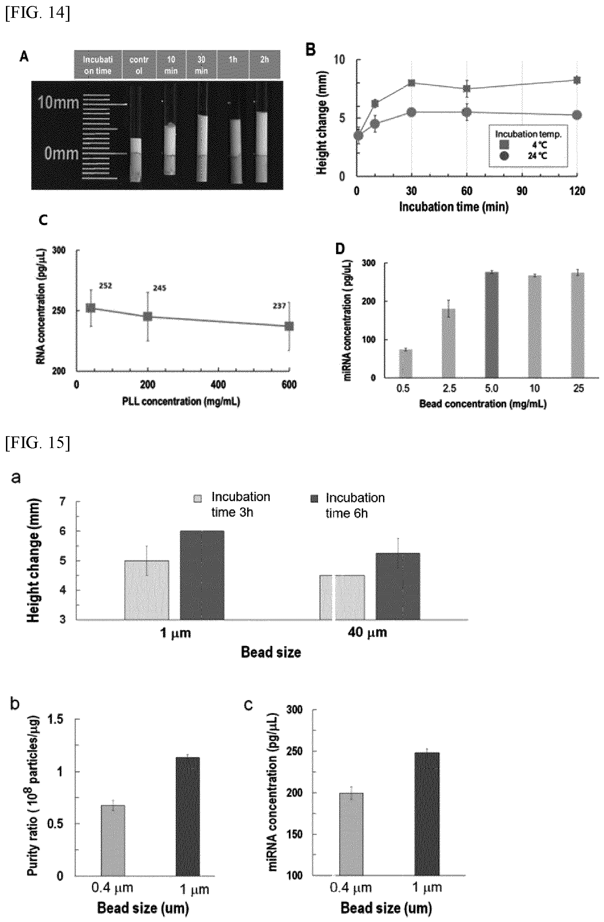

FIG. 14 shows the result of evaluating the performance of the PLL-magnetic beads based on a PLL concentration, an incubation temperature, and an incubation time.

Based on A in FIG. 14, it was identified that the volume of the PLL-magnetic bead was increased when 5 mg of the PLL-magnetic beads were added to 1 ml of the plasma sample and incubation thereon was performed while changing the incubation time to 10, 30, 60, and 120 minutes, respectively. B in FIG. 15 shows the performance as identified when the incubation time was 10 minutes, 30 minutes, 60 minutes, and 120 minutes and the incubation temperature was different temperatures of 4 °C and 24 °C, respectively.

As the volume of the PLL-magnetic bead increases, the binding between the PLL-magnetic bead and the extracellular vesicles contained in the plasma increases. 30 minutes of the incubation time seemed to have clearly better binding performance than the 10 minutes thereof has. There was no significant difference between the results of 30 minutes, 60 minutes and 120 minutes, so that the optimized condition was identified as 30 minutes. However, for a quick experiment, the 10 minutes incubation time may provide a not bad result.

Further, it was identified that the incubation temperature 4°C exhibited a better effect than the incubation temperature 24°C. Therefore, in this experiment, the incubation was performed at 4°C for 30 minutes.

C in FIG. 14 shows the result of identifying the effect of miRNA extraction when the PLL-magnetic beads are prepared by reacting the same bead with the PLL at varying concentrations of 40 mg/ml, 200 mg/ml, and 600 mg/ml, and the thus prepared PLL-magnetic beads are added to the same plasma sample. There was no significant difference in the overall PLL concentration when using the varying concentrations of 40 mg/ml, 200 mg/ml, and 600 mg/ml. However, the 40 mg/ml was found to be the most excellent. Therefore, in this experiment, the PLL-magnetic beads were prepared using the PLL concentration of 40 mg/ml.

D in FIG. 14 shows the results of identifying the miRNA extraction effect based on the concentration of the PLL-magnetic bead. The results were identified while changing the concentrations of the PLL-magnetic beads to 0.5 mg/ml, 2.5 mg/ml, 5 mg/ml, 10 mg/ml, and 25 mg/ml. it was identified that as the concentration of the PLL-magnetic beads increased up to 5 mg/ml, the miRNA extraction ability increased, whereas there was little change in the performance when the concentration was increased beyond 5 mg/ml. Therefore, this experiment was performed while the bead concentration was 5 mg/ml.

FIG. 15 shows the result of evaluating the extracellular vesicle isolation ability based on the size of the PLL-magnetic bead.

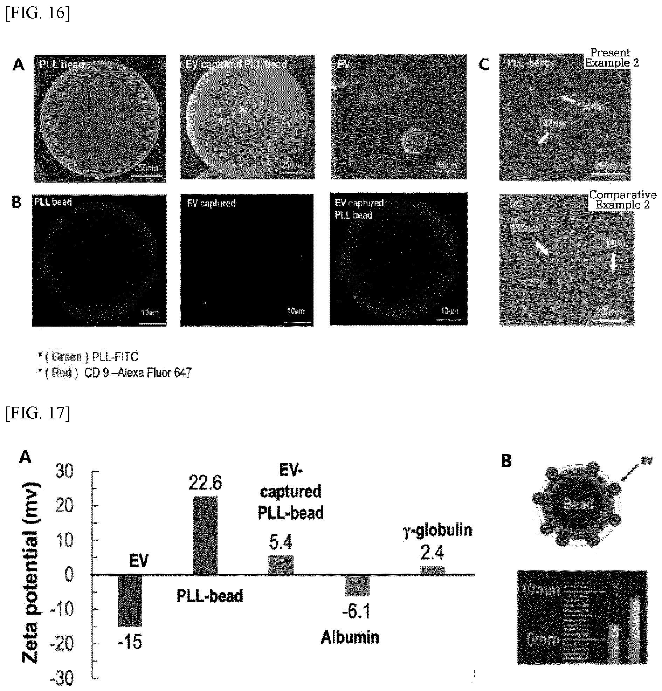

A in FIG. 15 shows the results of evaluation based on Hematocrit while the average diameter of the PLL-magnetic bead is each of 1 µm and 40 µm, and the incubation time is each of 3 hours and 6 hours. B in FIG. 16 shows the results of evaluation using a bioanalyzer device after setting the average diameter of the PLL-magnetic bead to each of 0.4 µm and 1 µm.

In a in FIG. 15, when the average diameter of the PLL-magnetic bead was 1 µm, the extracellular vesicle isolation performance was better than that when average diameter of the PLL-magnetic bead was 40 µm. The extracellular vesicle isolation performance was better when the incubation time was 6 hours than that when the incubation time was 3 hours. Further, in b of FIG. 15, it was identified that the case when the PLL-magnetic bead having an average diameter of 1 µm was used was superior, in terms of miRNA and the purity, to the case when the PLL-magnetic bead having an average diameter of 0.4 µm was used.

FIG. 16 shows the SEM image, the fluorescence image, and the cryo-TEM image according to each of Present Example 2 and Comparative Example 2.

A in FIG. 16 shows the result of isolation of extracellular vesicle (EV) using PLL-magnetic beads (PLL-bead) according to Present Example 2 and shows the SEM image of each of the PLL-magnetic bead, the PLL-magnetic bead having the extracellular vesicle captured thereon, and the isolated exosome. Based on the image, it was identified that the average diameter of the magnetic beads was 950 nm. B in FIG. 16 shows the fluorescence image of each of the PLL-magnetic bead (green color), the PLL-magnetic bead (red color) onto which exosomes are captured, and a combination thereof. C in FIG. 16 shows the cryo-TEM image of the extracellular vesicles (EVs) isolated according to each of Present Example 2 (PLL-bead) and Comparative Example 2 (UC).

In Present Example 2, the SEM images of PLL-magnetic beads of various sizes in a range of from 0.5 µm to 40 µm were identified and then the PLL-magnetic beads of 1 µm size were selected therefrom and then the evaluation was performed on the PLL-magnetic beads of the 1 µm size. It was identified that the PLL-magnetic beads were added to the plasma sample, and thereafter, the exosome as an extracellular vesicle was coated on the surface of the PLL-magnetic bead. Further, it was identified that the exosome isolated using the PLL-magnetic bead was approximately 100 nm in size.

It was identified that the results based on the fluorescence image were similar to those based on the SEM image. The PLL-magnetic beads were stained with FITC, and exosomes were stained with anti-CD 63 (Alexa Fluor 647) to identify fluorescence images. The fluorescence image was obtained using a 40 µm PLL-magnetic bead. Green indicates the PLL-magnetic bead, and red indicates the PLL-magnetic bead onto which the exosomes as an extracellular vesicle are captured. Based on the result of TEM-based identification of extracellular vesicles isolated using each of Present Example 2 (ExoCAS-2) and Comparative Example 2 (UC), it was identified that the extracellular vesicles of almost similar sizes in the range of 70 nm to 180 nm in diameter were isolated when using each of Present Example 2 and Comparative Example 2.

FIG. 17 shows the results of identifying the zeta potential and the volume change after incubation according to Present Example 2.

A in FIG. 17 shows the zeta potential of each of exosomes, Present Example 2 (PLL-bead), PPL-magnetic bead (EV-captured PLL-bead) with extracellular vesicles captured thereon, and albumin, γ-globulin and fibrinogen contained in the plasma protein. B in FIG. 17 shows the result of identifying the volume change before and after incubating Present Example 2 (PLL-bead) in the plasma.

The isolation of extracellular vesicles according to Present Example 2 (PLL-bead) uses anion exchange on the surface of the PLL-magnetic bead. The zeta potential of the exosome as an extracellular vesicle was -15.0 mV because an anionic phospholipid bilayer of the extracellular vesicle was formed. The PLL-magnetic bead exhibits a zeta potential of 22.6mV due to the cationic and hydrophilic amino group of the PLL. It was identified that when the exosome was captured on the surface of the PLL-magnetic bead, the zeta potential was reduced to 5.4 mV due to the binding of the exosome thereto. Further, the zeta potential of the PLL itself is 42.4mV.

That is, the zeta potential of PLL decreased to 22.6 mV while binding to the magnetic bead. The zeta potential of the PLL-magnetic bead was reduced to 5.4 mV due to the capture of the exosomes thereon. Proteins such as albumin, γ-globulin and fibrinogen were contained in the plasma protein. It was identified that they had negative values of zeta potentials of -6.1mV, 2.4mV and -18.7mV, respectively. Therefore, the protein having a negative zeta potential value together with the exosome as the extracellular vesicle may be captured onto the PLL-magnetic bead. Thus, a process of removing the impurities other than the exosome using the first reagent is required in the subsequent step.

It was identified that when the volumes of the PLL-magnetic bead before and after the incubation after adding the PLL-magnetic bead into the plasma were compared to each other, the volume change in the PLL-magnetic bead before and after the incubation occurred. It is determined that this is because the exosome is attached to the PLL-magnetic bead, and the attaching is controlled based on the incubation time and temperature. As reviewed in Present Example 1, it was identified that the attachment of exosomes to the PLL-magnetic bead was more effectively achieved in the incubation at 4°C than in the incubation at 24°C. In this experiment, it was identified that the volume was completely saturated after the incubation for 30 minutes.

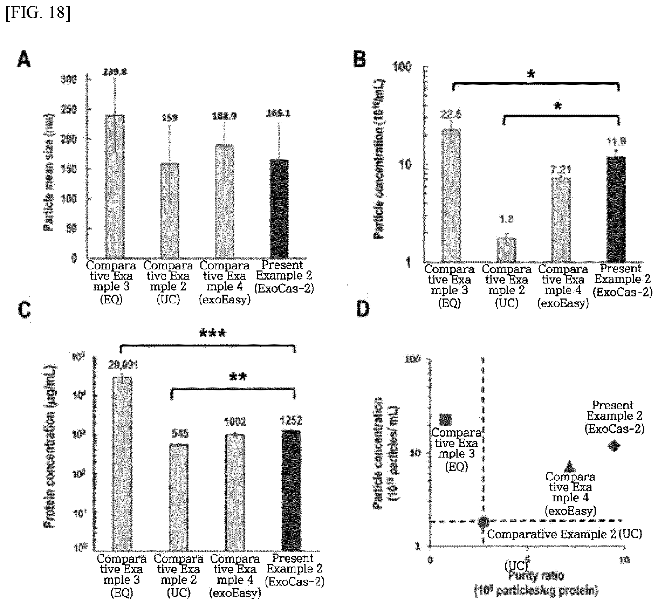

FIG. 18 shows the results of NTA and BCA analysis of each of Present Example 2 and Comparative Example 2 to Comparative Example 4.

FIG. 18 shows the average particle size (A), particle concentration (B), protein concentration (C), and purity ratio (a ratio of particle/protein) (D) regarding each of Present Example 2 and Comparative Example 2 to Comparative Example 4 (*: p <0.05, **: p <0.01, ***: p <0.001). In D of FIG. 18, the purity ratio of Comparative Example 2 was set as a reference.

We identified the size distribution, shape, surface, internal protein markers and exosome RNA of exosomes as the extracellular vesicle isolated using Present Example 2, which in turn were compared with those of exosomes as the extracellular vesicle isolated using each of the conventionally used ultra-centrifugation isolation of Comparative Example 2 (UC), Comparative Example 3 (EQ, exoQuick, SBI) and Comparative Example 4 (exoEasy).

It is determined that the sizes of exosomes isolated using Present Example 2, Comparative Example 2, and Comparative Example 4 except for Comparative Example 3 were similar to each other. To the contrary, there were differences between the particle concentrations and the protein contaminations of exosomes isolated using Present Example 2, Comparative Example 2, and Comparative Examples 3 and 4 (see B and C in FIG. 18).

In Comparative Example 3 having the isolation performance of exosomes, the maximum particle concentration was 22.5 x 1010/mL. This value was 12.5 times higher than 1.8 x 1010/mL in Comparative Example 2. The value in Present Example 2 was 11.9 x 1010/mL, which was about twice as high as that in Comparative Example 2. It may be identified that Comparative Example 3 exhibits a poor result in the protein contamination when compared with that in Comparative Example 2. When Comparative Example 2 and Present Example 2 are compared with each other, the protein contamination in Comparative Example 2 was approximately twice of that in Present Example 2. It was identified that the purity ratio defined as the ratio of particle concentration to protein concentration had the highest value in Present Example 2.

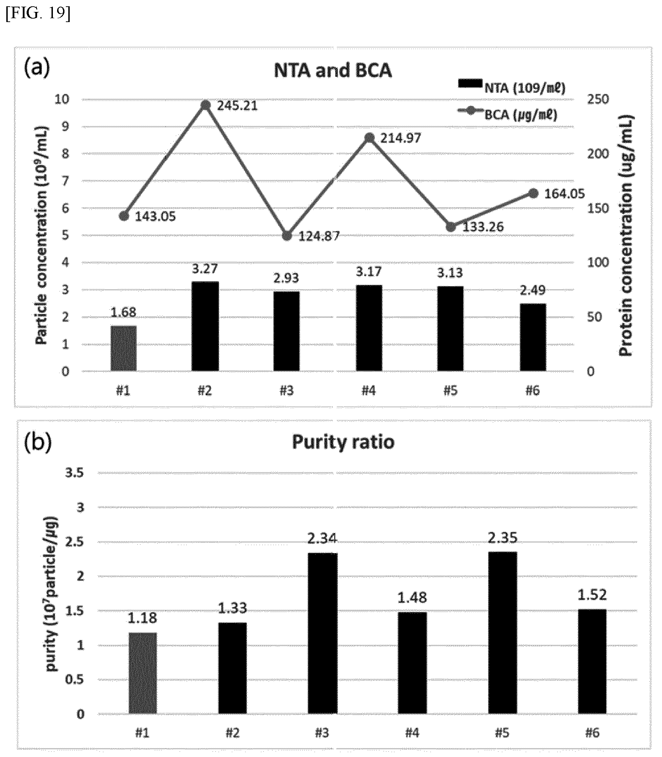

FIG. 19 shows the analysis results of NTA and BCA according to the first reagent of each of Present Example 2 and Present Example 3.

In FIG. 19, the first reagents in Present Example 2 and Present Example 3 were different from each other, and then, NaCl 1M at pH 7.0 (200 µL) was used as the second reagent, and the vortexing was executed for 5 minutes at 1000 rpm at room temperature. The ability of each of the first reagents in Present Example 2 and Present Example 3 to isolate the extracellular vesicles was evaluated. Table 1 below shows the conditions for washing using each of the first reagents. Table 2 shows the results of NTA, BTA, and purity (purity ratio) according to the conditions of the Table 1 below. In Table 1 below, the conditions of the first reagent include the pH and volume of the first reagent, and the first reagent (further) means washing the sample with the first reagent once and then additional washing (washing twice) of the same sample with the first reagent.

**[Table 1]**

| Examples | Sub-Examples | Temperature | First reagent | First reagent (further) |
|---|---|---|---|---|
| Present Example 2 | #1 | room temp. | pH 6 - 2mℓ | - |
| | #2 | room temp. | pH 6 - 2mℓ | - |
| | #3 | room temp. | pH 6 - 2mℓ | pH 6 - 2mℓ |
| Present Example 3 | #4 | room temp. | pH 6 - 4mℓ | - |
| | #5 | room temp. | pH 6 - 1mℓ | pH 8.5 - 1mℓ |
| | #6 | 70°C | pH 6 - 2mℓ | - |

**[Table 2]**

| Examples | Sub-Examples | NTA (10⁹/mℓ) | BCA (µg/mℓ) | Purity (10⁷ particle/µg) |
|---|---|---|---|---|
| Present Example 2 | #1 | 1.68 | 143.05 | 1.18 |
| Present Example 3 | #2 | 3.27 | 245.21 | 1.33 |
| | #3 | 2.93 | 124.87 | 2.34 |
| | #4 | 3.17 | 214.97 | 1.48 |
| | #5 | 3.13 | 133.26 | 2.35 |
| | #6 | 2.49 | 164.05 | 1.52 |

In (a) in FIG. 19, the particle concentration means the concentration of extracellular vesicles contained in the second reagent. A higher value thereof means that a larger amount of extracellular vesicles have been isolated. Thus, a higher value thereof means better isolation ability. The protein concentration means the concentration of the protein that acts as impurities rather than the extracellular vesicles contained in the second reagent. Thus, as the value thereof is lower, the first reagent has the better washing ability. In (b) in FIG. 19, this ability is indicated as the purity ratio (particle/protein ratio).

It was identified that when 2 ml of the first reagent of pH 6 was used under the same condition, Present Example 3 (#2) exhibited a better effect than Present Example 2 (#1) had. It was identified that when, as in Present Example 3, protamine salt was used as polyvalent cation, the isolation ability of extracellular vesicles was superior to that of PLL.

A result of comparing the cases of washing the same sample with the first reagents under different conditions (#2 to #6) of Present Example 3 with each other is as follows. It was identified that the case (#3) of washing the sample with the first reagent once and then additional washing (washing twice) of the same sample with the first reagent exhibited the better result than that in the case (#2) of washing the sample using the first reagent (2 ml) of pH 6.

Therefore, a result of comparing the case (#3) where the first reagent was washed twice (each time: 2 ml) and the case (#4) where the first reagent was washed once with 4 ml, which is twice of 2 ml with each other is as follows. The case (#3) has the better result than that in the case (#4).

Washing was performed twice using the first reagent (#3 and #5). #5 exhibited a similar result to that in #3 although a smaller volume of the first reagent was used in #5 than that in #3. Thus, it was identified that the isolation ability of extracellular vesicles was further improved, when the first reagent was used to wash the protein as the impurity as in #5, and when washing was performed with a weak base pH 6 first and then washing was further performed with a weak acid pH 8.5.

In order to identify the effect based on the temperature, an experiment on #6 was conducted. It was identified that the result thereof was almost similar to that of #2. Specifically, in the case of washing with the first reagent at the increased temperature, the particle concentration is decreased while the protein concentration is increased, so that the purity is generally similar to that of #2 conducted at room temperature. To the contrary, in the case of #6, the overall isolation efficiency is reduced compared to that in #2, because in the case of #6, it takes additional treatment and time such as the treatment of the first reagent in order to wash the sample at a high temperature, and maintaining the temperature in the process of washing.

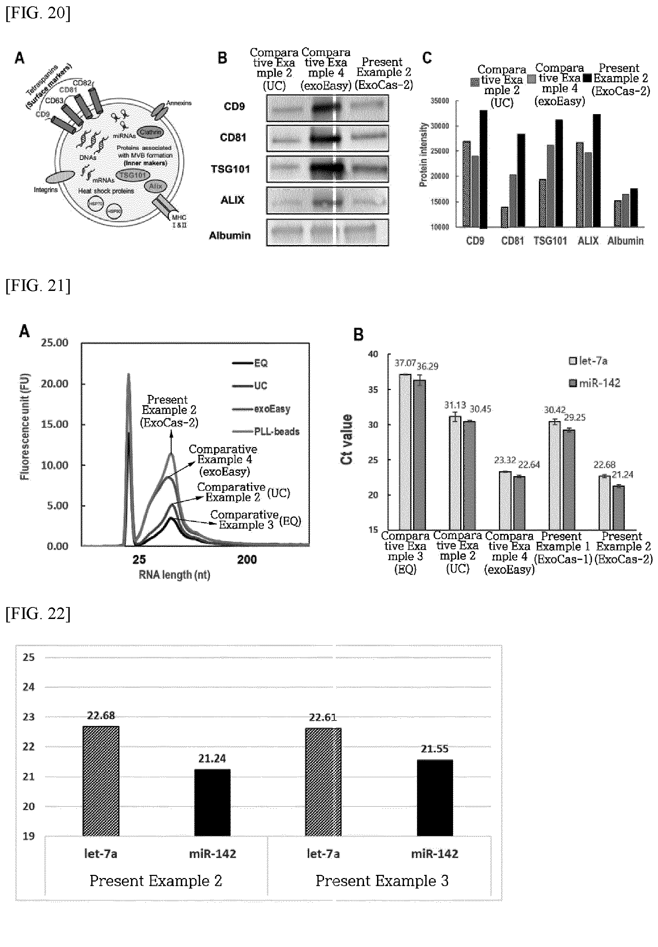

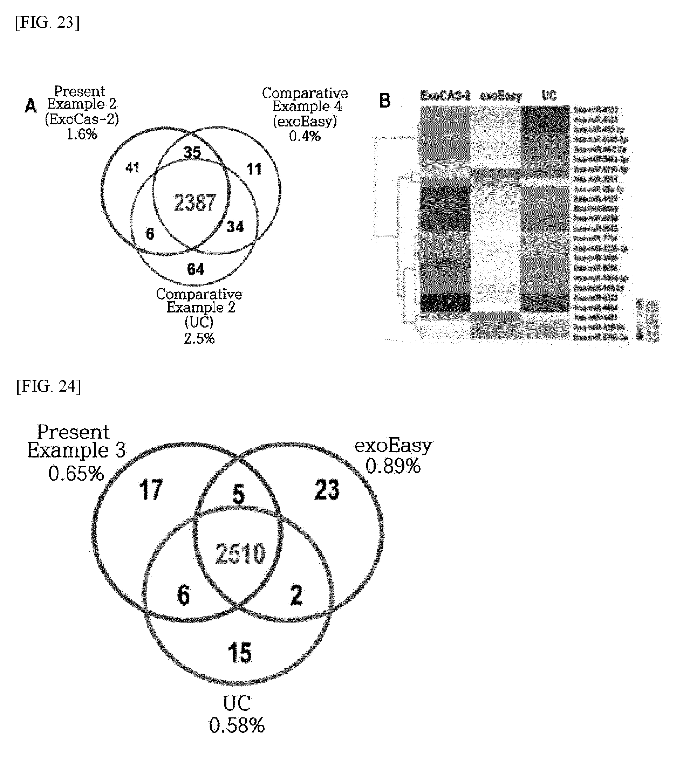

FIG. 20 shows the Western blot result of each of Present Example 2 and Comparative Example 2 to Comparative Example 4. FIG. 21 shows the performance evaluation result using a bioanalyzer of each of Present Example 2 and Comparative Example 2 to Comparative Example 4, and an exosome miRNA measurement result using RT-qPCR of each of Present Example 2 and Comparative Example 2 to Comparative Example 4. FIG. 22 shows the result of exosome miRNA measurement using RT-qPCR about each of Present Example 2 and Present Example 3. FIG. 23 is a graph depicting gene expression using a microarray according to each of Present Example 2 and Comparative Example 2 to Comparative Example 4. FIG. 24 is the result of using micro-array according to Present Example 3.

A and B in FIG. 20 show the results of identifying protein markers of extracellular vesicles using Western blot according to each of Present Example 2 and Comparative Example 2 to Comparative Example 4. C in FIG. 20 shows the analysis result of proteins extracted from extracellular vesicles using Western blot.

A in FIG. 21 shows the result of analyzing total exosome RNA with a bioanalysis device using an Agilent eukaryote total RNA pico chip according to each of Present Example 2 and Comparative Example 2 to Comparative Example 4. B in FIG. 21 shows the results of analysis of exosome miRNAs (hsa-let-7a-5p, hsa-miR-142-3p) as measured by RT-qPCR.

In FIG. 20, the presence of protein markers was investigated using Western blotting to identify whether the isolated extracellular vesicle is the exosome. Tetraspanins CD9 and CD81 often serve as exosome surface protein markers, and Alix (ALG-2-interacting protein X) and TSG101 (tumor susceptibility gene 101 protein) serve as protein markers inside exosomes. These four protein markers as reference protein markers for exosome identification were investigated. Further, in this experiment, albumin as a contaminating protein marker of the plasma protein was investigated

We could identify that Present Example 2 among Present Example 2, Comparative Example 2, and Comparative Example 4 (except Comparative Example 3 because of its relatively poor performance) exhibited strong bands in the reference protein markers (ALIX, TSG101, CD9 and CD81). Additional quantitative analysis was performed on the band intensity (B in FIG. 20).

The intensity of the exosome protein marker was as follows: Present Example 2 > Comparative Example 2 > Comparative Example 4. It was identified that, based on the surface protein markers (CD9 and CD81) and the internal protein markers (TSG101 and ALIX), the isolated extracellular vesicle was an exosome. That is, the extracellular vesicle isolated according to Present Example 2 of the present disclosure is the exosome.

In all of Present Example 2, Comparative Example 2, and Comparative Example 4, the band intensity of albumin occurred, wherein the band intensity of albumin was low, and the band intensities of albumin in Present Example 2, Comparative Example 2, and Comparative Example 4 were similar to each other.

Referring to A of FIG. 21, the exosomes were isolated from the same plasma using each of Present Example 2 and Comparative Example 2 to Comparative Example 4. RNA of the exosomes was extracted using SeraMir Exosome RNA Purification Kit (SBI, Palo Alto, CA, USA). After the RNA extraction, the total RNA was measured using an Agilent Bioanalyzer 2100 (Agilent Technologies, Santa Clara, CA, USA). Present Example 2 exhibited the highest total RNA value, and Comparative Example 3 exhibited the lowest value.

Referring to B in FIG. 21, the portion of RNA extracted from the exosome was analyzed using RT-qPCR. The Ct values of the two types of miRNAs exhibited similar values in the same isolation method. However, it was identified that there were significant differences between the values obtained using the respective isolation methods, that is, Present Example 2 and Comparative Example 2 to Comparative Example 4. Comparative Example 2 exhibited 31.1 and 30.5 for hsa-let-7a-5p and hsa-miR-142-3p, respectively. Comparative Example 3 exhibited high values of 37.07 and 36.29 for hsa-let-7a-5p and hsa-miR-142-3p, respectively, which may be considered to be due to high protein contamination. Present Example 2 exhibited the lowest Ct values of 22.7 and 21.2 for hsa-let-7a-5p and hsa-miR-142-3p, respectively. Comparative Example 4 exhibited 23.3 and 22.6 for hsa-let-7a-5p and hsa-miR-142-3p, respectively.

Referring to FIG. 22, it may be identified that the Ct values of the two types of miRNAs in Present Example 2 and Present Example 3 are similar to each other.

Referring to A of FIG. 23, it was identified that, based on a result of evaluation using Gen-Chip miRNA 4.0 array (Affymetrix Inc., Santa Clara, CA, USA), a total of 2578 miRNAs was identified in the extracellular vesicles extracted using each of Present Example 2, Comparative Example 2, and Comparative Example 4. It was identified that in all of Present Example 2, Comparative Example 2, and Comparative Example 4, 92.6% of exosomal miRNAs was observed.

Referring to B of FIG. 23, it was identified that 26 miRNA genes exhibiting differences in expressions thereof was free of a specific miRNA related to cancer. It was identified that a percentage of miRNA identified using each of Present Example 2, Comparative Example 2, and Comparative Example 4 did not exceed 2.5%.

Referring to FIG. 23 and FIG. 24, it may be identified that Present Example 3 exhibits 0.65 % and thus has approximately similar performance to that of each of Comparative Example 2 and Comparative Example 4 as conventionally used.

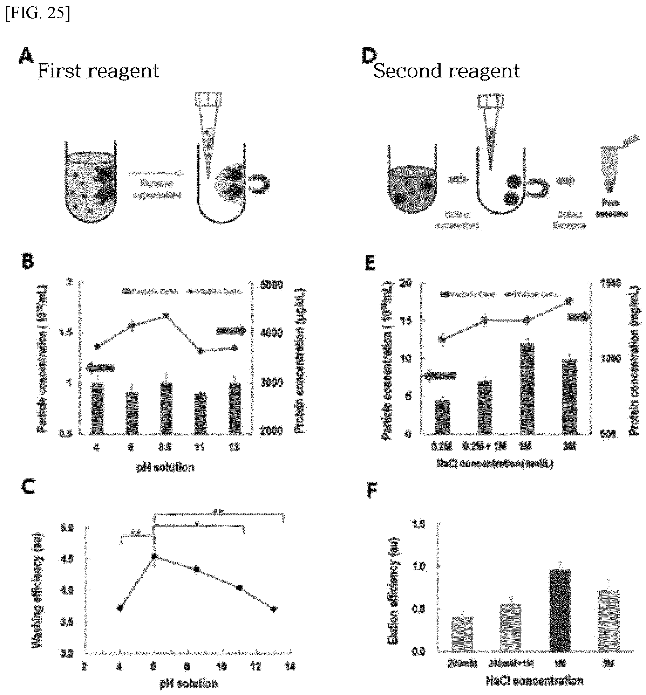

FIG. 25 shows the results of performance evaluation in the step (a) of using the first reagent and the step (b) of using the second reagent in Present Example 2.

A to C of FIG. 23 show the particle concentration, protein concentration and washing efficiency (protein/particle ratio) respectively as a result of washing and removing impurities other than extracellular vesicles from the PPL-magnetic beads using the first reagent after the PPL-magnetic beads were added to the plasma to prepare with PPL-magnetic bead onto which the extracellular vesicles are captured. D to F in FIG. 22 show the particle concentration, protein concentration, and washing efficiency (protein/particle ratio), respectively (* : p <0.05, ** : p < 0.01) as the result of the elution of the PPL-magnetic beads onto which the extracellular vesicle are captured using the second reagent.

Referring to FIG. 23, the PPL-magnetic beads of Present Example 2 are based on the principle of anion exchange, and as shown in A in FIG. 22, were washed with the first reagent to remove the impurities, that is, the proteins attached to the PPL-magnetic beads. In this regard, the negatively charged proteins should be removed while the removal does not affect the extracellular vesicles captured onto the PPL-magnetic bead. Since the pH of the first reagent is equal to the isoelectric point (pI), and the protein charge becomes neutral, the protein may be easily isolated therefrom via the ion exchange resin. Further, the pH of the second reagent may be adjusted to a value below the isoelectric point, such that a positive charge of the protein may be induced. However, the isoelectric point of the plasma proteins is in a wide range of from 5 to 9. This causes a problem.

Using the varying pH of the first reagent, the ability thereof to remove the impurities attached to the PPL-magnetic bead was identified (B in FIG. 22). In the first reagent, the pH thereof was changed by variously changing the sodium hydroxide concentration (6.135 nM) while the acetic acid concentration (29 nM) was kept constant.

As a result, it was identified that the concentration of the protein exhibited the maximum value at pH 8.5, while the concentration of the particles was almost the same at the varying connections. The washing efficiency defined as the ratio between the total number of particles as washed and the concentration of the protein washed by the first reagent was the highest when the first reagent had pH 6.

The extracellular vesicles captured onto the PPL-magnetic beads were isolated using the second reagent (D in FIG. 22). After experiments were executed using various salts (Na₂SO₄, (NH₄)₂SO₄, KCl, CH₃COONH₄ and NaCl), it was identified that the anion exchange ability of sodium chloride exhibited an appropriate result. Thus, the sodium chloride was used as the salt of the second reagent.

NaCl of various concentrations (in a range of 200mM to 3M) and concentration changes (200mM and 1M) were used to prepare the second reagents. Then, an amount by which the extracellular vesicles captured onto the PPL-magnetic beads were eluted therefrom based on the various concentrations (in a range of 200mM to 3M) and concentration changes (200mM and 1M) was identified.

In E of FIG. 25, it was identified that the amount of the collected particles increased as the NaCl concentration increased, such that the value had a maximum value at 1M, and then decreased again at 3M. It is determined that this is because the salting-out effect occurred. On the contrary, the concentration of the protein contained in the second reagent with which the elution was performed increased as the NaCl concentration increased.

That is, after the elution of the extracellular vesicles captured onto the PPL-magnetic beads therefrom using the second reagent, the elution effect defined as the ratio of the number of particles to the concentration of the eluted protein may be measured. It was identified that the elution effect had the highest value when the concentration of NaCl was 1M, and at this time, a significant amount of the exosomes was extracted.

The embodiments as described above are not restrictive but illustrative in all respects. The scope of the present invention is indicated by the following claims rather than the above detailed descriptions.

### Reference numerals

100: Adsorbent sphere
110: Support
120: Polyvalent cation

## Claims

1. A microvesicles isolation method to isolate microvesicles contained in the biological sample from the sample, the method comprising:
(a) adding an adsorbent sphere to the biological sample containing the microvesicles therein;
(b) keeping the adsorbent sphere in the biological sample to form an adsorbent sphere conjugate composed of the adsorbent sphere and the microvesicles captured thereon;
(c) isolating the adsorbent sphere conjugate from the biological sample;
(d) washing the isolated adsorbent sphere conjugate using a first reagent, including washing the adsorbent sphere having the microvesicles captured thereon under a first condition using the first reagent,
wherein the first reagent contains at least one of CH₃COO⁻, SO₄²⁻, HCO⁻, SiO⁻, and OH⁻,
wherein the first condition is that the washing is carried out at least once in a range of pH 5.5 to 6.5 or pH 7.5 to 9.5; and
(e) eluting the microvesicles from the washed adsorbent sphere conjugate using a second reagent,
wherein the adsorbent sphere includes a support, and one or more polyvalent cations disposed on a surface of the support.

2. The microvesicles isolation method of claim 1, wherein the biological sample includes at least one of blood, plasma, serum, urine, saliva, cerebrospinal fluid, tears, sweat, feces, ascites, amniotic fluid, semen, milk, cell medium, tissue extract and cancer tissue.

3. The microvesicles isolation method of claim 1 or 2, wherein the polyvalent cation includes at least one of polylysine, protamine, polyarginine, polyhistidine, cationic dextran, cationic dendrimer, cationic polysaccharide, polyamidoamine, polyethyleneimine, polyquaternium, poly-2-dimethylaminoethyl methacrylate (PDMAEMA), poly (2-dimethylaminomethyl styrene) (PDMAMS), poly-1-vinylpyrrolidone (p1-VP), poly diethylaminoethyl acrylate (pDEAEA), poly dimethylaminoethyl acrylate (pDMAEA), poly diethylaminoethyl methacrylate (pDEAMA), lipopolyamines, quaternary ammoniums, guanidine, imidazole, polyaniline, polypyrrol, or chitosan.

4. The microvesicles isolation method of claim 1, wherein the support includes at least one of a porous particle, a porous membrane, or a porous mesh.

5. The microvesicles isolation method of claim 1, wherein the support further includes irregularities formed on the surface thereof.

6. The microvesicles isolation method of claim 1, wherein the support is a magnetic bead containing at least one metal selected from a group consisting of iron (Fe), nickel (Ni), cobalt (Co), manganese (Mn), bismuth (Bi) and zinc (Zn), or an alloy thereof, or a metal oxide or alloy oxide of a metal selected from the group.

7. The microvesicles isolation method of claim 1, wherein the support includes a core as an inner portion thereof, and a shell provided to surround an outer face of the core,
wherein the core includes at least one of polyacrylate, polyacrylamide, polymethacrylate, polyethylene glycol, polystyrene vinylbenzene, polystyrene, hydrogel, agarose, ceramic, silica gel, or latex,
wherein the shell contains at least one metal selected from a group consisting of iron (Fe), nickel (Ni), cobalt (Co), manganese (Mn), bismuth (Bi) and zinc (Zn), or an alloy thereof, or a metal oxide or alloy oxide of a metal selected from the group,
wherein the polyvalent cation includes at least one of poly-L-lysine polymer (PLL), protamine, quaternary ammoniums, chitosan, or polyhistidine.

8. The microvesicles isolation method of claim 1, wherein the step (e) includes eluting the microvesicles from the washed adsorbent sphere conjugate using the second reagent under a second condition,
wherein the second reagent contains at least one of CH₃COO⁻, SO₄²⁻, Cl⁻, HCO⁻, SiO⁻, and OH⁻ at a concentration range of 0.2 M to 3 M,
wherein the second condition is that the elution is performed at least once via mixing at 500rpm to 2000rpm and for 1 minute to 60 minutes.

9. The microvesicles isolation method of claim 1, wherein the method further comprises:
after washing the adsorbent sphere conjugate with the first reagent in the step (d), performing a first auxiliary isolation to isolate the adsorbent sphere conjugate; and
after the elution of the microvesicles with the second reagent in the step (e), performing a second auxiliary isolation to isolate the adsorbent sphere,
wherein the support is embodied as a bead having a circular cross section and made of at least one of polyacrylate, polyacrylamide, polymethacrylate, polyethylene glycol, polystyrene vinylbenzene, or polystyrene,
wherein the first auxiliary isolation or the second auxiliary isolation is performed using a capturing filter having pores of 50 nm to 1,000 nm in size.

10. The microvesicles isolation method of claim 1, wherein the method further comprises:
after washing the adsorbent sphere conjugate with the first reagent in the step (d), performing a first auxiliary isolation to isolate the adsorbent sphere conjugate; and
after the elution of the microvesicles with the second reagent in the step (e), performing a second auxiliary isolation to isolate the adsorbent sphere,
wherein the support includes a magnetic particle,
wherein the first auxiliary isolation or the second auxiliary isolation includes magnetic isolation to cause current to flow in an electromagnet to generate a magnetic force or using a magnet.

11. The microvesicles isolation method of claim 1, wherein the microvesicles is coupled to the adsorbent sphere via at least one of electrostatic interaction or intercalation,
wherein a zeta potential of the adsorbent sphere conjugate is in a range of 0.5mV to 10mV.

12. A reagent kit for microvesicles isolation configured to isolate the microvesicles contained in a biological sample, the reagent kit comprising:
one or more adsorbent spheres having binding ability to the microvesicle;
a first reagent containing at least one of CH₃COO⁻, SO₄²⁻, HCO⁻, SiO⁻, and OH⁻ and having a pH in the range of pH 5.5 to 6.5 or pH 7.5 to 9.5; and
a second reagent containing at least one of CH₃COO⁻, SO₄²⁻, Cl⁻, HCO⁻, SiO⁻, and OH⁻ ,
wherein the adsorbent sphere includes a support, and one or more polyvalent cations disposed on a surface of the support.

13. The reagent kit of claim 12, wherein the support is embodied as a bead having a circular cross section and made of at least one of polyacrylate, polyacrylamide, polymethacrylate, polyethylene glycol, polystyrene vinylbenzene, or polystyrene,
wherein the polyvalent cation includes at least one of poly-L-lysine polymer (PLL), protamine, quaternary ammoniums, chitosan, or polyhistidine,
wherein the isolation member includes a capturing filter having pores of 50 nm to 1,000 nm in size.

14. The reagent kit of claim 12, wherein the support includes a magnetic particle,
wherein the polyvalent cation includes at least one of poly-L-lysine polymer (PLL), protamine, quaternary ammoniums, chitosan, or polyhistidine,
wherein the isolation member performs magnetic isolation using a device to cause a current to flow in an electromagnet to generate a magnetic force or using a magnet.

## Patentansprüche

1. Mikrovesikelisolierungsverfahren zum Isolieren von Mikrovesikeln, die in der biologischen Probe enthalten sind, aus der Probe, wobei das Verfahren Folgendes umfasst:
(a) Hinzufügen einer Adsorptionskugel zu der biologischen Probe, die die Mikrovesikel darin enthält;
(b) Halten der Adsorptionskugel in der biologischen Probe, um ein Adsorptionskugelkonjugat zu bilden, das aus der Adsorptionskugel und den daran festgehaltenen Mikrovesikeln besteht;
(c) Isolieren des Adsorptionskugelkonjugats von der biologischen Probe;
(d) Waschen des isolierten Adsorptionskugelkonjugats unter Verwendung eines ersten Reagenzes, einschließlich Waschen der Adsorptionskugel mit den daran festgehaltenen Mikrovesikeln unter einer ersten Bedingung unter Verwendung des ersten Reagenzes,
wobei das erste Reagenz mindestens eines von CH₃COO⁻, SO₄²⁻, HCO⁻, SiO⁻ und OH⁻ enthält,
wobei die erste Bedingung daraus besteht, dass das Waschen mindestens einmal in einem Bereich von pH 5,5 bis 6,5 oder pH 7,5 bis 9,5 ausgeführt wird; und
(e) Eluieren der Mikrovesikel aus dem gewaschenen Adsorptionskugelkonjugat unter Verwendung eines zweiten Reagenzes,
wobei die Adsorptionskugel einen Träger und ein oder mehrere polyvalente Kationen, die auf einer Oberfläche des Trägers angeordnet sind, beinhaltet.

2. Mikrovesikelisolierungsverfahren nach Anspruch 1, wobei die biologische Probe mindestens eines von Blut, Plasma, Serum, Urin, Speichelflüssigkeit, Rückenmarksflüssigkeit, Tränen, Schweiß, Kot, Bauchwasser, Fruchtwasser, Samen, Milch, Zellmedium, Gewebeextrakt und Krebsgewebe beinhaltet.

3. Mikrovesikelisolierungsverfahren nach Anspruch 1 oder 2, wobei das polyvalente Kation mindestens eines von Polylysin, Protamin, Polyarginin, Polyhistidin, kationischem Dextran, kationischem Dendrimer, kationischem Polysaccharid, Polyamidoamin, Polyethylenimin, Polyquaternium, Poly-2-dimethylaminoethylmethacrylat (PDMAEMA), Poly(2-dimethylaminomethylstyrol) (PDMAMS), Poly-1-vinylpyrrolidon (pl-VP), Polydiethylaminoethylacrylat (pDEAEA), Polydimethylaminoethylacrylat (pDMAEA), Polydiethylaminoethylmethacrylat (pDEAMA), Lipopolyaminen, quaternären Ammoniumverbindungen, Guanidin, Imidazol, Polyanilin, Polypyrrol oder Chitosan beinhaltet.

4. Mikrovesikelisolierungsverfahren nach Anspruch 1, wobei der Träger mindestens eines von einem porösen Teilchen, einer porösen Membran oder einem porösen Netz beinhaltet.

5. Mikrovesikelisolierungsverfahren nach Anspruch 1, wobei der Träger ferner Unregelmäßigkeiten beinhaltet, die auf der Oberfläche davon gebildet sind.

6. Mikrovesikelisolierungsverfahren nach Anspruch 1, wobei der Träger ein Magnetkügelchen ist, das mindestens ein Metall, das ausgewählt ist aus einer Gruppe bestehend aus Eisen (Fe), Nickel (Ni), Cobalt (Co), Mangan (Mn), Wismut (Bi) und Zink (Zn) oder einer Legierung davon, oder ein Metalloxid oder ein Legierungsoxid eines Metalls, das aus der Gruppe ausgewählt ist, enthält.

7. Mikrovesikelisolierungsverfahren nach Anspruch 1, wobei der Träger einen Kern als einen inneren Abschnitt davon und eine Hülle, die bereitgestellt ist, um eine äußere Fläche des Kerns zu umgeben, beinhaltet,
wobei der Kern mindestens eines von Polyacrylat, Polyacrylamid, Polymethacrylat, Polyethylenglycol, Polystyrolvinylbenzol, Polystyrol, Hydrogel, Agarose, Keramik, Kieselgel oder Latex beinhaltet,
wobei die Hülle mindestens ein Metall, das ausgewählt ist aus einer Gruppe bestehend aus Eisen (Fe), Nickel (Ni), Cobalt (Co), Mangan (Mn), Wismut (Bi) und Zink (Zn) oder einer Legierung davon, oder ein Metalloxid oder ein Legierungsoxid eines Metalls, das aus der Gruppe ausgewählt ist, enthält,
wobei das polyvalente Kation mindestens eines von Poly-L-Lysin-Polymer (PLL), Protamin, quaternären Ammoniumverbindungen, Chitosan oder Polyhistidin beinhaltet.

8. Mikrovesikelisolierungsverfahren nach Anspruch 1, wobei der Schritt (e) Eluieren der Mikrovesikel aus dem gewaschenen Adsorptionskugelkonjugat unter Verwendung des zweiten Reagenzes unter einer zweiten Bedingung beinhaltet,
wobei das zweite Reagenz mindestens eines von CH₃COO⁻, SO₄²⁻, Cl⁻, HCO⁻, SiO⁻ und OH⁻ in einem Konzentrationsbereich von 0,2 M bis 3 M enthält,
wobei die zweite Bedingung ist, dass die Elution mindestens einmal durch Mischen bei 500 U/min bis 2000 U/min und für 1 Minute bis 60 Minuten durchgeführt wird.

9. Mikrovesikelisolierungsverfahren nach Anspruch 1, wobei das Verfahren ferner Folgendes umfasst:
nach dem Waschen des Adsorptionskugelkonjugats mit dem ersten Reagenz in dem Schritt (d), Durchführen einer ersten Hilfsisolierung, um das Adsorptionskugelkonjugat zu isolieren; und
nach der Elution der Mikrovesikel mit dem zweiten Reagenz in dem Schritt (e), Durchführen einer zweiten Hilfsisolierung, um die Adsorptionskugel zu isolieren,
wobei der Träger als ein Kügelchen mit einem kreisförmigen Querschnitt verkörpert ist und aus mindestens einem von Polyacrylat, Polyacrylamid, Polymethacrylat, Polyethylenglycol, Polystyrolvinylbenzol oder Polystyrol besteht,
wobei die erste Hilfsisolierung oder die zweite Hilfsisolierung unter Verwendung eines Auffangfilters mit Poren mit einer Größe von 50 nm bis 1000 nm durchgeführt wird.

10. Mikrovesikelisolierungsverfahren nach Anspruch 1, wobei das Verfahren ferner Folgendes umfasst:
nach dem Waschen des Adsorptionskugelkonjugats mit dem ersten Reagenz in dem Schritt (d), Durchführen einer ersten Hilfsisolierung, um das Adsorptionskugelkonjugat zu isolieren; und
nach der Elution der Mikrovesikel mit dem zweiten Reagenz in dem Schritt (e), Durchführen einer zweiten Hilfsisolierung, um die Adsorptionskugel zu isolieren,
wobei der Träger ein magnetisches Teilchen beinhaltet,
wobei die erste Hilfsisolierung oder die zweite Hilfsisolierung magnetische Isolierung, um zu bewirken, dass Strom in einem Elektromagnet fließt, um eine magnetische Kraft zu erzeugen, oder das Verwenden eines Magneten beinhaltet.

11. Mikrovesikelisolierungsverfahren nach Anspruch 1, wobei die Mikrovesikel über mindestens eine von einer elektrostatischen Interaktion oder Interkalation an die Adsorptionskugel gekoppelt werden,
wobei ein Zeta-Potential des Adsorptionskugelkonjugats in einem Bereich von 0,5 mV bis 10 mV liegt.

12. Reagenzkit zur Mikrovesikelisolierung, das dazu konfiguriert ist, die Mikrovesikel, die in einer biologischen Probe enthalten sind, zu isolieren, wobei das Reagenzkit Folgendes umfasst:
eine oder mehrere Adsorptionskugeln mit Bindungsfähigkeit an das Mikrovesikel;
ein erstes Reagenz, das mindestens eines von CH₃COO⁻, SO₄²⁻, HCO⁻, SiO⁻ und OH⁻ enthält und einen pH-Wert im Bereich von pH 5,5 bis 6,5 oder pH 7,5 bis 9,5 aufweist; und
ein zweites Reagenz, das mindestens eines von CH₃COO⁻, SO₄²⁻, Cl⁻, HCO⁻, SiO⁻ und OH⁻ enthält,
wobei die Adsorptionskugel einen Träger und ein oder mehrere polyvalente Kationen, die auf einer Oberfläche des Trägers angeordnet sind, beinhaltet.

13. Reagenzkit nach Anspruch 12, wobei der Träger als ein Kügelchen mit einem kreisförmigen Querschnitt verkörpert ist und aus mindestens einem von Polyacrylat, Polyacrylamid, Polymethacrylat, Polyethylenglycol, Polystyrolvinylbenzol oder Polystyrol besteht,
wobei das polyvalente Kation mindestens eines von Poly-L-Lysin-Polymer (PLL), Protamin, quaternären Ammoniumverbindungen, Chitosan oder Polyhistidin beinhaltet,
wobei das Isolierungselement einen Auffangfilter mit Poren mit einer Größe von 50 nm bis 1000 nm beinhaltet.

14. Reagenzkit nach Anspruch 12, wobei der Träger ein magnetisches Teilchen beinhaltet,
wobei das polyvalente Kation mindestens eines von Poly-L-Lysin-Polymer (PLL), Protamin, quaternären Ammoniumverbindungen, Chitosan oder Polyhistidin beinhaltet,
wobei das Isolierungselement magnetische Isolierung unter Verwendung einer Vorrichtung, um zu bewirken, dass ein Strom in einem Elektromagnet fließt, um eine magnetische Kraft zu erzeugen, oder unter Verwendung eines Magneten durchführt.

## Revendications

1. Procédé d'isolement de microvésicules pour isoler des microvésicules contenues dans l'échantillon biologique à partir de l'échantillon, le procédé comprenant :
(a) l'ajout d'une sphère adsorbante à l'échantillon biologique contenant les microvésicules ;
(b) le maintien de la sphère adsorbante dans l'échantillon biologique pour former un conjugué de sphère adsorbante composé de la sphère adsorbante et des microvésicules capturées sur celle-ci ;
(c) l'isolement du conjugué de sphère adsorbante à partir de l'échantillon biologique ;
(d) le lavage du conjugué de sphère adsorbante isolé à l'aide d'un premier réactif, incluant le lavage de la sphère adsorbante comportant les microvésicules capturées sur celle-ci dans une première condition à l'aide du premier réactif,
dans lequel le premier réactif contient au moins un parmi CH₃COO⁻, SO₄²⁻, HCO⁻, SiO⁻ et OH⁻,
dans lequel la première condition est que le lavage soit réalisé au moins une fois dans une plage de pH 5,5 à 6,5 ou de pH 7,5 à 9,5 ; et
(e) l'élution des microvésicules à partir du conjugué de sphère adsorbante lavé, à l'aide d'un deuxième réactif,
dans lequel la sphère adsorbante inclut un support, et un ou plusieurs cations polyvalents disposés sur une surface du support.

2. Procédé d'isolement de microvésicules selon la revendication 1, dans lequel l'échantillon biologique inclut au moins un parmi : sang, plasma, sérum, urine, salive, liquide céphalo-rachidien, larmes, sueur, matières fécales, ascite, liquide amniotique, sperme, lait, milieu cellulaire, extrait tissulaire et tissu cancéreux.

3. Procédé d'isolement de microvésicules selon la revendication 1 ou 2, dans lequel le cation polyvalent inclut au moins un parmi : polylysine, protamine, polyarginine, polyhistidine, dextrane cationique, dendrimère cationique, polysaccharide cationique, polyamidoamine, polyéthylèneimine, polyquaternium, poly-2-diméthylaminoéthyl méthacrylate (PDMAEMA), poly (2-diméthylaminométhyl styrène) (PDMAMS), poly-1-vinylpyrrolidone (p1-VP), poly diéthylaminoéthyl acrylate (pDEAEA), poly diméthylaminoéthyl acrylate (pDMAEA), poly diéthylaminoéthyl méthacrylate (pDEAMA), lipopolyamines, ammoniums quaternaires, guanidine, imidazole, polyaniline, polypyrrole ou chitosane.

4. Procédé d'isolement de microvésicules selon la revendication 1, dans lequel le support inclut au moins une particule poreuse, une membrane poreuse ou un maillage poreux.

5. Procédé d'isolement de microvésicules selon la revendication 1, dans lequel le support inclut en outre des irrégularités formées sur sa surface.

6. Procédé d'isolement de microvésicules selon la revendication 1, dans lequel le support est une bille magnétique contenant au moins un métal choisi dans le groupe consistant en : fer (Fe), nickel (Ni), cobalt (Co), manganèse (Mn), bismuth (Bi) et zinc (Zn), ou un de leurs alliages, ou un oxyde métallique ou un oxyde d'alliage d'un métal choisi dans le groupe.

7. Procédé d'isolement de microvésicules selon la revendication 1, dans lequel le support inclut un noyau comme portion interne de celui-ci, et une enveloppe prévue pour entourer une face externe du noyau,
dans lequel le noyau inclut au moins un parmi : polyacrylate, polyacrylamide, polyméthacrylate, polyéthylène glycol, polystyrène vinylbenzène, polystyrène, hydrogel, agarose, céramique, gel de silice ou latex,
dans lequel l'enveloppe contient au moins un métal choisi dans un groupe consistant en : fer (Fe), nickel (Ni), cobalt (Co), manganèse (Mn), bismuth (Bi) et zinc (Zn), ou un de leurs alliages, ou un oxyde métallique ou un oxyde d'alliage d'un métal choisi dans le groupe,
dans lequel le cation polyvalent inclut au moins un parmi : polymère poly-L-lysine (PLL), protamine, ammoniums quaternaires, chitosane ou polyhistidine.

8. Procédé d'isolement de microvésicules selon la revendication 1, dans lequel l'étape (e) inclut l'élution des microvésicules à partir du conjugué de sphère adsorbante lavé, à l'aide du deuxième réactif dans une deuxième condition,
dans lequel le deuxième réactif contient au moins un parmi : CH₃COO⁻, SO₄²⁻, Cl⁻, HCO⁻, SiO⁻ et OH⁻ à une plage de concentration de 0,2 M à 3 M,
dans lequel la deuxième condition est que l'élution soit réalisée au moins une fois par mélange à 500 tr/min à 2000 tr/min pendant 1 minute à 60 minutes.

9. Procédé d'isolement de microvésicules selon la revendication 1, dans lequel le procédé comprend en outre :
après lavage du conjugué de sphère adsorbante avec le premier réactif à l'étape (d), la réalisation d'un premier isolement auxiliaire pour isoler le conjugué de sphère adsorbante ; et
après l'élution des microvésicules avec le deuxième réactif à l'étape (e), la réalisation d'un deuxième isolement auxiliaire pour isoler la sphère adsorbante,
dans lequel le support est construit sous la forme d'une bille ayant une coupe circulaire et constituée d'au moins un parmi : polyacrylate, polyacrylamide, polyméthacrylate, polyéthylène glycol, polystyrène vinylbenzène, ou polystyrène,
dans lequel le premier isolement auxiliaire ou le deuxième isolement auxiliaire est réalisé à l'aide d'un filtre de capture ayant des pores d'une taille de 50 nm à 1000 nm.

10. Procédé d'isolement de microvésicules selon la revendication 1, dans lequel le procédé comprend en outre :
après lavage du conjugué de sphère adsorbante avec le premier réactif à l'étape (d), la réalisation d'un premier isolement auxiliaire pour isoler le conjugué de sphère adsorbante ; et
après l'élution des microvésicules avec le deuxième réactif à l'étape (e), la réalisation d'un deuxième isolement auxiliaire pour isoler la sphère adsorbante,
dans lequel le support inclut une particule magnétique,
dans lequel le premier isolement auxiliaire ou le deuxième isolement auxiliaire inclut une isolation magnétique pour faire circuler un courant dans un
électroaimant afin de générer une force magnétique, ou l'utilisation d'un aimant.

11. Procédé d'isolement de microvésicules selon la revendication 1, dans lequel les microvésicules sont couplées à la sphère adsorbante via au moins une parmi une interaction électrostatique ou une intercalation,
dans lequel un potentiel zêta du conjugué de sphère adsorbante est dans une plage de 0,5 mV à 10 mV.

12. Kit de réactifs pour l'isolement de microvésicules, configuré pour isoler des microvésicules contenues dans un échantillon biologique, le kit de réactifs comprenant :
une ou plusieurs sphères adsorbantes ayant une capacité de liaison avec la microvésicule ;
un premier réactif contenant au moins un parmi : CH₃COO⁻, SO₄²⁻, HCO⁻, SiO⁻ et OH⁻ et ayant un pH dans la plage de pH 5,5 à 6,5 ou de pH 7,5 à 9,5 ; et
un deuxième réactif contenant au moins un parmi : CH₃COO⁻, SO₄²⁻, Cl⁻, HCO⁻, SiO⁻ et OH⁻,
dans lequel la sphère adsorbante inclut un support, et un ou plusieurs cations polyvalents disposés sur une surface du support.

13. Kit de réactifs selon la revendication 12, dans lequel le support est construit sous la forme d'une bille ayant une coupe circulaire et constituée d'au moins un parmi : polyacrylate, polyacrylamide, polyméthacrylate, polyéthylène glycol, polystyrène vinylbenzène, ou polystyrène,
dans lequel le cation polyvalent inclut au moins un parmi : polymère poly-L-lysine (PLL), protamine, ammoniums quaternaires, chitosane ou polyhistidine,
dans lequel l'organe d'isolement inclut un filtre de capture ayant des pores d'une taille de 50 nm à 1000 nm.

14. Kit de réactifs selon la revendication 12, dans lequel le support inclut une particule magnétique,
dans lequel le cation polyvalent inclut au moins un parmi : polymère poly-L-lysine (PLL), protamine, ammoniums quaternaires, chitosane ou polyhistidine,
dans lequel l'organe d'isolement réalise une isolation magnétique à l'aide d'un dispositif pour faire circuler un courant dans un électroaimant afin de générer une force magnétique, ou à l'aide d'un aimant.
